(19) Europäisches Patentamt
European Patent Office
Office européen des brevets

(11) EP 2 998 293 A1

(12) **EUROPEAN PATENT APPLICATION**
published in accordance with Art. 153(4) EPC

(43) Date of publication:
**23.03.2016 Bulletin 2016/12**

(21) Application number: **14797515.5**

(22) Date of filing: **16.05.2014**

(51) Int Cl.:
***C07D 209/70*** *(2006.01)* ***C07D 409/04*** *(2006.01)*
***C07D 417/04*** *(2006.01)* ***H01L 51/05*** *(2006.01)*
***H01L 51/30*** *(2006.01)* ***H01L 51/46*** *(2006.01)*

(86) International application number:
**PCT/JP2014/063127**

(87) International publication number:
**WO 2014/185536 (20.11.2014 Gazette 2014/47)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **16.05.2013 JP 2013104472**
**02.10.2013 JP 2013207724**

(71) Applicants:
- **Daikin Industries, Ltd.**
  **Osaka-shi, Osaka 530-8323 (JP)**
- **Osaka University**
  **Suita-shi, Osaka 565-0871 (JP)**

(72) Inventors:
- **NAGAI, Takabumi**
  **Osaka-shi**
  **Osaka 530-8323 (JP)**
- **ADACHI, Kenji**
  **Osaka-shi**
  **Osaka 530-8323 (JP)**
- **ASO, Yoshio**
  **Suita-shi**
  **Osaka 565-0871 (JP)**
- **IE, Yutaka**
  **Suita-shi**
  **Osaka 565-0871 (JP)**
- **KARAKAWA, Makoto**
  **Suita-shi**
  **Osaka 565-0871 (JP)**

(74) Representative: **Hoffmann Eitle**
**Patent- und Rechtsanwälte PartmbB**
**Arabellastraße 30**
**81925 München (DE)**

# (54) FULLERENE DERIVATIVE AND N-TYPE SEMICONDUCTOR MATERIAL

(57) An object of the present invention is to provide a material that exhibits excellent properties as an n-type semiconductor, in particular as an n-type semiconductor for use in a photoelectric conversion element, such as organic thin-film solar cells. The present invention relates to a fullerene derivative represented by formula (1):

EP 2 998 293 A1

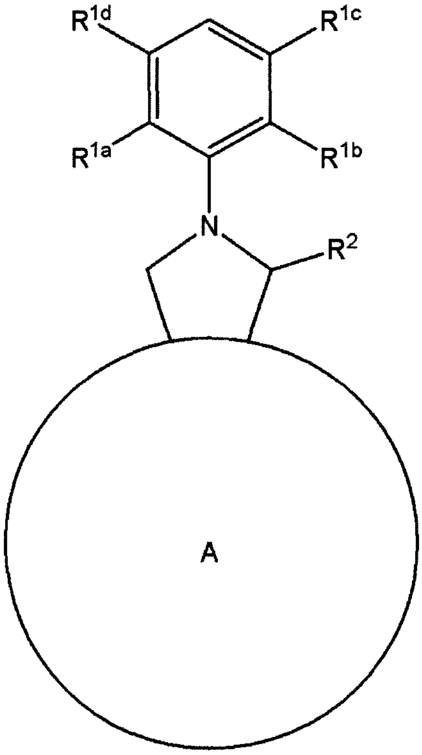

(1)

wherein
$R^{1a}$ and $R^{1b}$ are the same or different, and each represents a hydrogen atom or a fluorine atom;
$R^{1c}$ and $R^{1d}$ are the same or different, and each represents a hydrogen atom, a fluorine atom, alkyl, alkoxy, ester, or cyano;
$R^2$ represents (1) phenol optionally substituted with at least one substituent selected from the group consisting of fluorine, alkyl, alkoxy, ester, and cyano, or (2) a 5-membered heteroaryl group optionally substituted with 1 to 3 methyl groups; and ring A represents a fullerene ring,
with the proviso that when $R^{1a}$, $R^{1b}$, $R^{1c}$, and $R^{1d}$ are each a hydrogen atom, $R^2$ represents phenyl substituted with 1 or 2 fluorine atoms or a 5-membered heteroaryl group optionally substituted with 1 to 3 methyl groups.

## Description

### Technical Field

The present invention relates to a fullerene derivative, an n-type semiconductor material, and the like.

### Background Art

Organic thin-film solar cells are formed by a coating technique with a solution of an organic compound, which is a photoelectric conversion material. The cells have various advantages: for example, 1) device production cost is low; 2) area expansion is easy; 3) the cells are more flexible than inorganic materials, such as silicon, thus enabling a wider range of applications; and 4) resource depletion is less likely. As such, organic thin-film solar cells have been developed, and the use of the bulk heterojunction structure has particularly led to a significant increase in conversion efficiency, thus attracting widespread attention.

For p-type semiconductor of the photoelectric conversion basic materials used for organic thin-film solar cells, poly-3-hexylthiophene (P3HT) is particularly known as an organic p-type semiconductor material exhibiting excellent performance. With an aim to obtain advanced materials, recent developments have provided compounds (donor-acceptor type n-conjugated polymers) that can absorb broad wavelengths of solar light or that have tuned energy levels, leading to significant improvements in the performance. Examples of such compounds include poly-p-phenylenevinylene and poly[[4,8-bis[(2-ethylhexyl)oxy]benzo[1,2-b:4,5-b']dithiophene-2,6-diyl][3-fluoro-2-[(2-ethylhexyl)carbonyl]thieno[3,4-b]thiophenediyl]] (PTB7).

For n-type semiconductors as well, fullerene derivatives have been intensively studied, and [6,6]-phenyl-$C_{61}$-butyric acid methyl ester (PCBM) has been reported as a material having excellent photoelectric conversion performance (see the below-listed Patent Documents 1, 2, etc.). Nonetheless, there have been few reports that demonstrate stable and excellent conversion efficiency of fullerene derivatives except for PCBM.

Although fullerene derivatives for organic solar cells other than PCBM have been reported, the reports concern a comparison using special devices from which a power collection material of the positive electrode (ITO electrode) is removed (Non-patent Document 1), or fullerene derivatives only showing performance almost equivalent to that of PCBM (Non-patent Document 2). Although the disubstituted derivatives reported by Y. Li et al. (Non-patent Document 3), when used with P3TH, achieved higher conversion efficiency than PCBM as reported by E. T. Hoke et al., the disubstituted derivatives exhibited only low conversion efficiency when used with a donor-acceptor n-conjugated polymer (Non-patent Document 4).

Thus, except for PCBM, advanced n-type materials capable of achieving high conversion efficiency, independently of p-type materials, have been unknown.

Several methods for synthesizing fullerene derivatives have been proposed. Methods known to be excellent from the standpoint of yield and purity include a method for synthesizing, using a diazo compound, a fullerene derivative having a 3-membered ring moiety and a method for synthesizing a fullerene derivative having a 5-membered ring moiety to which an azomethine ylide generated from a glycine derivative and an aldehyde is added.

The aforementioned PCBM is a fullerene derivative having a 3-membered ring moiety , and PCBM can be obtained by preparing a mixture of three types of products each having a fullerene backbone to which a carbene intermediate is added, and subjecting the mixture to a conversion reaction by light irradiation or heat treatment. However, the derivative having a 3-membered ring moiety obtained by this production method is restricted in terms of the introduction site of substituent and the number of substituents; thus, the development of novel n-type semiconductors has significant limitations.

Fullerene derivatives having a 5-membered ring moiety, on the other hand, are considered to be excellent because of their diverse structures. However, there have been few reports on the fullerene derivatives having excellent performance as an n-type semiconductor material for organic thin-film solar cells. One of a few examples is the fullerene derivative disclosed in the below-listed Patent Document 3.

### Citation List

#### Patent Documents

Patent Document 1: JP2009-084264A
Patent Document 2: JP2010-092964A
Patent Document 3: JP2012-089538A

Non-patent Documents

Non-patent Document 1: T. Itoh et al., Journal of Materials Chemistry, 2010, vol. 20, page 9,226
Non-patent Document 2: T. Ohno et al., Tetrahedron, 2010, vol. 66, page 7,316
Non-patent Document 3: Y. Li et al., Journal of American Chemical Society, 2010, vol. 132, page 1,377
Non-patent Document 4: E.T. Hoke et al., Advanced Energy Materials, 2013, vol. 3, page 220

Summary of Invention

Technical Problem

The present invention has been completed in view of the above-described status quo of the related art, and the major object is to provide a material having excellent performance as an n-type semiconductor, more specifically as an n-type semiconductor for photoelectric conversion elements such as organic thin-film solar cells.

Solution to Problem

Patent Document 3 states that the fullerene derivative disclosed in the document shows high photoelectric conversion efficiency. A study conducted by the present inventors suggested that the basicity of the amine in the pyrrolidine moiety contained in the fullerene derivative is the major factor in this high photoelectric conversion efficiency.

A further study by the present inventors led to the following new findings: the sterically bulky structure of the substituent at position 2 of the pyrrolidine moiety affects the performance of the fullerene derivative as an n-type semiconductor; more specifically, a derivative having a more bulky substituent exhibits decreased conversion efficiency. However, the study also revealed that when the pyrrolidine moiety is not substituted at position 2, the fullerene derivative shows low solubility, and low conversion efficiency.

The present inventors conducted extensive research on the basis of these findings, and found that a fullerene derivative represented by formula (1) below has excellent performance as an n-type semiconductor.

The present invention provides a fullerene derivative represented by formula (1) below, and an n-type semiconductor material and the like consisting of the fullerene derivative.

Item 1.

A fullerene derivative represented by formula (1):

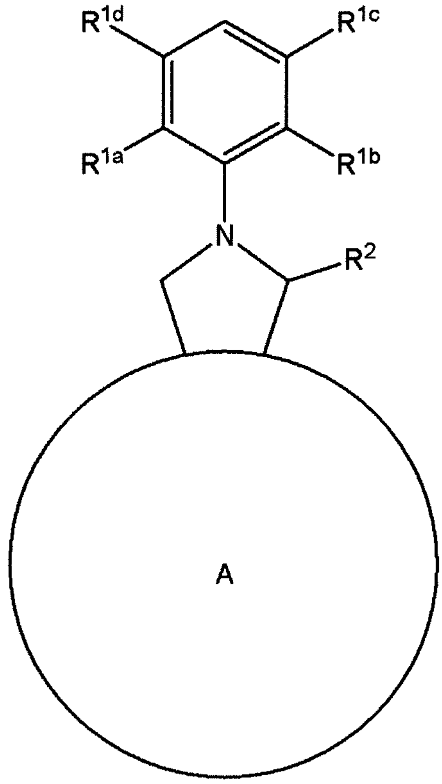

(1)

wherein

$R^{1a}$ and $R^{1b}$ are the same or different, and each represents a hydrogen atom or a fluorine atom;
$R^{1c}$ and $R^{1d}$ are the same or different, and each represents a hydrogen atom, a fluorine atom, alkyl optionally substituted with at least one fluorine atom, alkoxy optionally substituted with at least one fluorine atom, ester, or cyano;
$R^2$ represents

(1) phenyl optionally substituted with at least one substituent selected from the group consisting of fluorine, alkyl, alkoxy, ester, and cyano,
(2) a 5-membered heteroaryl group optionally substituted with 1 to 3 methyl groups, or
(3) alkyl, alkoxy, ether, acyl, ester, or cyano; and

ring A represents a fullerene ring;

with the proviso that when $R^{1a}$, $R^{1b}$, $R^{1c}$, and $R^{1d}$ are each a hydrogen atom, $R^2$ represents phenyl substituted with 1 or 2 fluorine atoms or a 5-membered heteroaryl group optionally substituted with 1 to 3 methyl groups.

Item 2.

The fullerene derivative according to Item 1, wherein
$R^{1a}$ and $R^{1b}$ are the same or different, and each represents a hydrogen atom or a fluorine atom;
at least one of $R^{1a}$ and $R^{1b}$ is a fluorine atom; and $R^2$ is a group represented by the following formula:

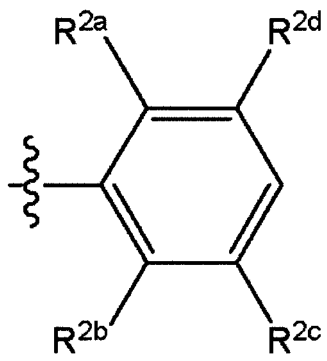

wherein,

$R^{2a}$ and $R^{2b}$ are the same or different, and each represents a hydrogen atom, a fluorine atom, alkyl, or alkoxy; and $R^{2c}$ and $R^{2d}$ are the same or different, and each represents a hydrogen atom, a fluorine atom, alkyl, alkoxy, ester, or cyano.

Item 3.

The fullerene derivative according to Item 2, wherein
$R^{1a}$ and $R^{1b}$ are the same or different, and each represents a hydrogen atom or a fluorine atom;
at least one of $R^{1a}$ and $R^{1b}$ is a fluorine atom;
$R^{1c}$ and $R^{1d}$ are the same or different, and each represents a hydrogen atom or a fluorine atom;
$R^{2a}$ and $R^{2b}$ are the same or different, and each represents a hydrogen atom, a fluorine atom, alkyl, or alkoxy; and $R^{2c}$ and $R^{2d}$ each represents a hydrogen atom.

Item 4.

The fullerene derivative according to any one of Items 1 to 3, wherein the ring A is $C_{60}$ fullerene or $C_{70}$ fullerene.

Item 5.

An n-type semiconductor material consisting of the fullerene derivative according to any one of Items 1 to 4.

Item 6.

The n-type semiconductor material according to Item 5, which is for use in an organic thin-film solar cell.

Item 7.

An organic power-generating layer comprising the n-type semiconductor material according to Item 6.

Item 8.

A photoelectric conversion element comprising the organic power-generating layer according to Item 7.

Item 9.

The photoelectric conversion element according to Item 8, which is an organic thin-film solar cell.

Item 10.

The n-type semiconductor material according to Item 5, which is for use in a photosensor array.

Item 11

The photoelectric conversion element according to Item 8, which is for use in a photosensor array.

Advantageous Effects of Invention

The fullerene derivative according to the present invention is useful as an n-type semiconductor material, particularly an n-type semiconductor for photoelectric conversion elements such as organic thin-film solar cells.

Description of Embodiments

As used herein, "alkyl" refers to a linear or branched $C_{1-10}$ alkyl such as methyl, ethyl, propyl, isopropyl, butyl, isobutyl, sec-butyl, tert-butyl, pentyl, isopentyl, neopentyl, and hexyl, unless indicated otherwise.

As used herein, "alkoxy" refers to, for example, a group represented by RO- wherein R is alkyl, unless indicated otherwise.

As used herein, "ester" refers to, for example, a group represented by $RCO_2$- wherein R is alkyl, unless indicated otherwise.

As used herein, "ether" refers to a group having an ether bond (-O-), and includes a polyether group, unless indicated otherwise. The polyether group includes a group represented by formula: $R^a$-(O-$R^b$)$_n$- wherein $R^a$ is alkyl, $R^b$ is the same or different in each occurrence, and is alkylene, and n is an integer of 1 or more. The alkylene is a divalent group formed by removing one hydrogen atom from the above-described alkyl).

As used herein, "acyl" includes alkanoyl, unless indicated otherwise. As used herein, "alkanoyl" refers to, for example, a group represented by RCO- wherein R is alkyl, unless indicated otherwise.

As used herein, a "5-membered heteroaryl group" refers to, for example, a 5-membered heteroaryl group containing as members of its ring at least one heteroatom (e.g., 1, 2, or 3 heteroatoms) selected from the group consisting of oxygen, sulfur, and nitrogen, unless indicated otherwise; examples of the 5-membered heteroaryl group include pyrrolyl (e.g., 1-pyrrolyl, 2-pyrrolyl, and 3-pyrrolyl), furil (e.g., 2-furil, and 3-furil), thienyl (e.g., 2-thienyl, and 3-thienyl), pyrazolyl (e.g., 1-pyrazolyl, 3-pyrazolyl, and 4-pyrazolyl), imidazolyl (e.g., 1-imidazolyl, 2-imidazolyl, and 4-imidazolyl), isoxazolyl (e.g., 3-isoxazolyl, 4-isoxazolyl, and 5-isoxazolyl), oxazolyl (e.g., 2-oxazolyl, 4-oxazolyl, and 5-oxazolyl), isothiazolyl (e.g., 3-isothiazolyl, 4-isothiazolyl, and 5-isothiazolyl), thiazolyl (e.g., 2-thiazolyl, 4-thiazolyl, and 5-thiazolyl), triazolyl (e.g., 1,2,3-triazole-4-yl, and 1,2,4-triazole-3-yl), oxadiazolyl (e.g., 1,2,4-oxadiazole-3-yl, and 1,2,4-oxadiazole-5-yl), and thiadiazolyl (e.g., 1,2,4-thiadiazole-3-yl, and 1,2,4-thiadiazole-5-yl).

The following describes in detail a fullerene derivative according to the present invention, an n-type semiconductor material, and the like consisting of).

Fullerene Derivative

The fullerene derivative according to the present invention is represented by the following formula (1)

Formula (1):

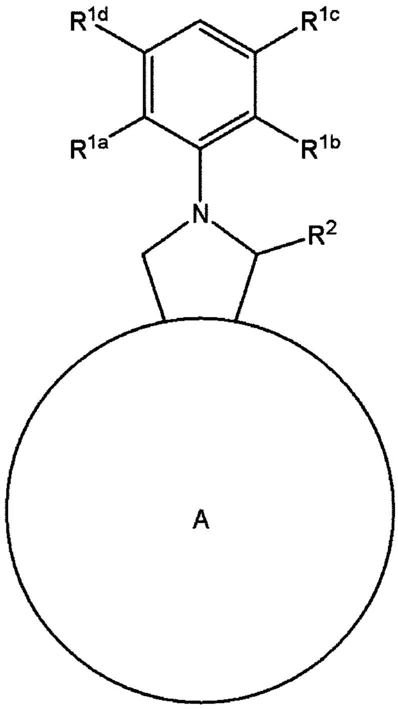

(1)

wherein

$R^{1a}$ and $R^{1b}$ are the same or different, and each represents a hydrogen atom or a fluorine atom;
$R^{1c}$ and $R^{1d}$ are the same or different, and each represents a hydrogen atom, a fluorine atom, alkyl optionally substituted with at least one fluorine atom, alkoxy optionally substituted with at least one fluorine atom, ester, or cyano;
$R^2$ represents

(1) phenyl optionally substituted with at least one substituent selected from the group consisting of fluorine, alkyl, alkoxy, ester, and cyano,
(2) a 5-membered heteroaryl group optionally substituted with 1 to 3 methyl groups, or
(3) alkyl, alkoxy, ether, acyl, ester, or cyano; and

ring A represents a fullerene ring;

with the proviso that when $R^{1a}$, $R^{1b}$, $R^{1c}$, and $R^{1d}$ are each a hydrogen atom, $R^2$ represents phenyl substituted with 1 or 2 fluorine atoms or a 5-membered heteroaryl group optionally substituted with 1 to 3 methyl groups.

The fullerene derivative according to the present invention has a group represented by the following partial structural formula,

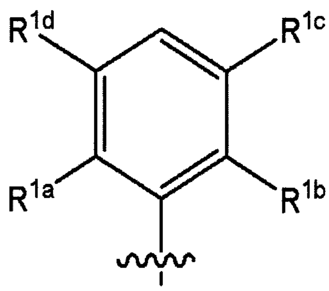

which is attached to the nitrogen atom, a constituent atom of the pyrrolidine moiety in formula (1), wherein the symbols are as defined above. This weakens the base property attributable to the nitrogen atom, thereby providing excellent properties as an n-type semiconductor material.

Preferable examples of the group include 2-fluorophenyl and 2,6-difluorophenyl.

$R^2$ is preferably a group represented by the following formula:

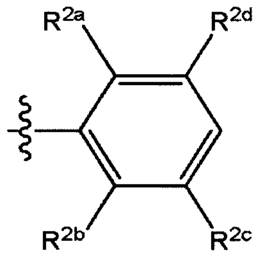

wherein $R^{2a}$ and $R^{2b}$ are the same or different, and each represents a hydrogen atom, a fluorine atom, alkyl, or alkoxy; and

$R^{2c}$ and $R^{2d}$ are the same or different, and each represents a hydrogen atom, a fluorine atom, alkyl, alkoxy, ester, or cyano.

Preferable examples of $R^2$ include 2-fluorophenyl, 2,6-difluorophenyl, 2-methoxyphenyl, and 2,6-dimethoxyphenyl.

In a preferable embodiment of the present invention, $R^{1a}$ and $R^{1b}$ are the same or different, and each represents a hydrogen atom or a fluorine atom; at least one of $R^{1a}$ and $R^{1b}$ is a fluorine atom; and $R^2$ is a group represented by the following formula:

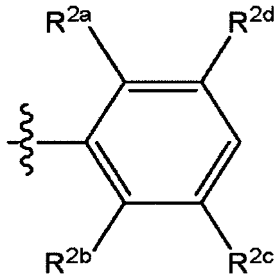

wherein,

$R^{2a}$ and $R^{2b}$ are the same or different, and each represents a hydrogen atom, a fluorine atom, alkyl, or alkoxy; and $R^{2c}$ and $R^{2d}$ are the same or different, and each represents a hydrogen atom, a fluorine atom, alkyl, alkoxy, ester, or cyano.

In the embodiment, more preferably, $R^{1a}$ and $R^{1b}$ are the same or different, each represents a hydrogen atom or a fluorine atom, and at least one of $R^{1a}$ and $R^{1b}$ is a fluorine atom; $R^{1c}$ and $R^{1d}$ are the same or different, and each represents a hydrogen atom or a fluorine atom; $R^{2a}$ and $R^{2b}$ are the same or different, and each represents a hydrogen atom, a fluorine atom, alkyl, or alkoxy; and $R^{2c}$ and $R^{2d}$ are each a hydrogen atom.

Ring A is preferably $C_{60}$ fullerene or $C_{70}$ fullerene, and more preferably $C_{60}$ fullerene.

The fullerene derivative represented by formula (1) may be a mixture of a fullerene derivative having $C_{60}$ fullerene as ring A and a fullerene derivative having $C_{70}$ fullerene as ring A. As used herein, $C_{60}$ fullerene may be represented by the following structural formula, which is often used in this technical field:

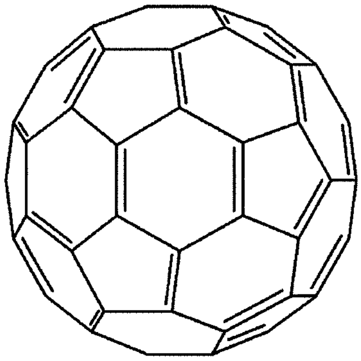

When ring A is $C_{60}$ fullerene, the fullerene derivative of formula (1) can be represented by the following formula.

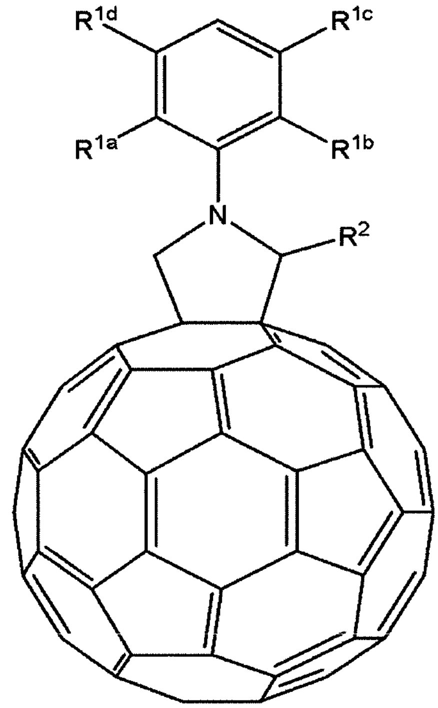

A fullerene derivative of one embodiment of the present invention is represented by the following formula (1A):

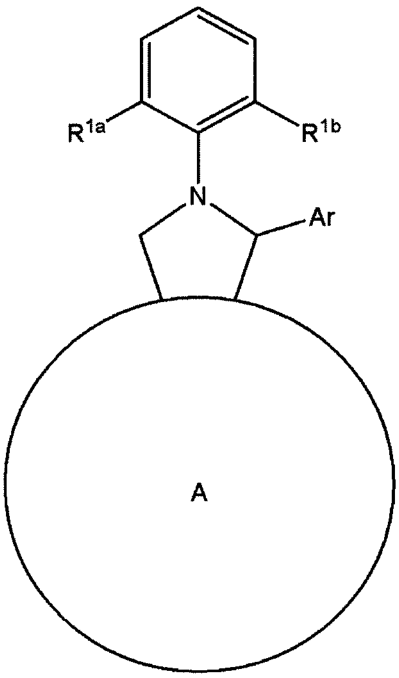

(1A)

wherein,

$R^{1a}$ and $R^{1b}$ are the same or different, and each represents a hydrogen atom or a fluorine atom;
Ar is phenyl optionally substituted with 1 or 2 fluorine atoms, or a 5-membered heteroaryl group optionally substituted with 1 to 3 methyl groups; and
ring A represents a fullerene ring,

with the proviso that when $R^{1a}$ and $R^{1b}$ are both hydrogen atoms, Ar is phenyl substituted with 1 or 2 fluorine atoms, or a 5-membered heteroaryl group optionally substituted with 1 to 3 methyl groups.

The fullerene derivative in this embodiment has a compact, substituted or unsubstituted phenyl (i.e., phenyl, 2-fluorophenyl, or 2,6-difluorophenyl) represented by the following partial structural formula,

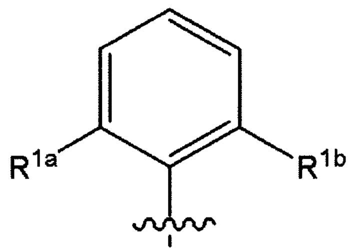

which is attached to the nitrogen atom, a constituent atom of the pyrrolidine moiety in formula (1A). This weakens the base property attributable to the nitrogen atom, thereby providing excellent properties as an n-type semiconductor material.

In this embodiment, Ar is preferably phenyl substituted with 1 or 2 fluorine atoms, or a 5-membered heteroaryl group optionally substituted with 1 to 3 methyl groups.

Because Ar is such a compact, substituted or unsubstituted aromatic group, the fullerene derivative of the present invention can exhibit excellent properties as an n-type semiconductor material.

In this embodiment, the "phenyl substituted with 1 or 2 fluorine atoms" represented by Ar is preferably phenyl substituted with 1 or 2 fluorine atoms at the ortho position (i.e., 2-fluorophenyl, or 2,6-difluorophenyl).

In this embodiment, preferable examples of Ar include phenyl, 2-fluorophenyl, 2,6-difluorophenyl, 2-thienyl, and 2-thiazolyl, and more preferable examples include phenyl, 2-fluorophenyl, and 2,6-difluorophenyl.

In a preferable embodiment of the fullerene derivative represented by formula (1A), at least one of $R^{1a}$ and $R^{1b}$ is a fluorine atom.

In another preferable embodiment of the fullerene derivative represented by formula (1A), $R^{1a}$ and $R^{1b}$ are both

hydrogen atoms, and
Ar is phenyl substituted with 1 or 2 fluorine atoms, or a 5-membered heteroaryl group optionally substituted with 1 to 3 methyl groups.

Because the fullerene derivative represented by formula (1) shows excellent solubility in various organic solvents, it is easy to form a thin film using a coating technique.

In addition, the fullerene derivative represented by formula (1) easily forms a bulk heterojunction structure, when used as an n-type semiconductor material to prepare an organic power-generating layer, together with an organic p-type semiconductor material.

Method for Producing a Fullerene Derivative

The fullerene derivative represented by formula (1) can be produced by a known method for producing a fullerene derivative, or by a method complying therewith.

Specifically, the fullerene derivative represented by formula (1) can be synthesized, for example, in accordance with the following scheme. The symbols indicated in the scheme are as defined above.

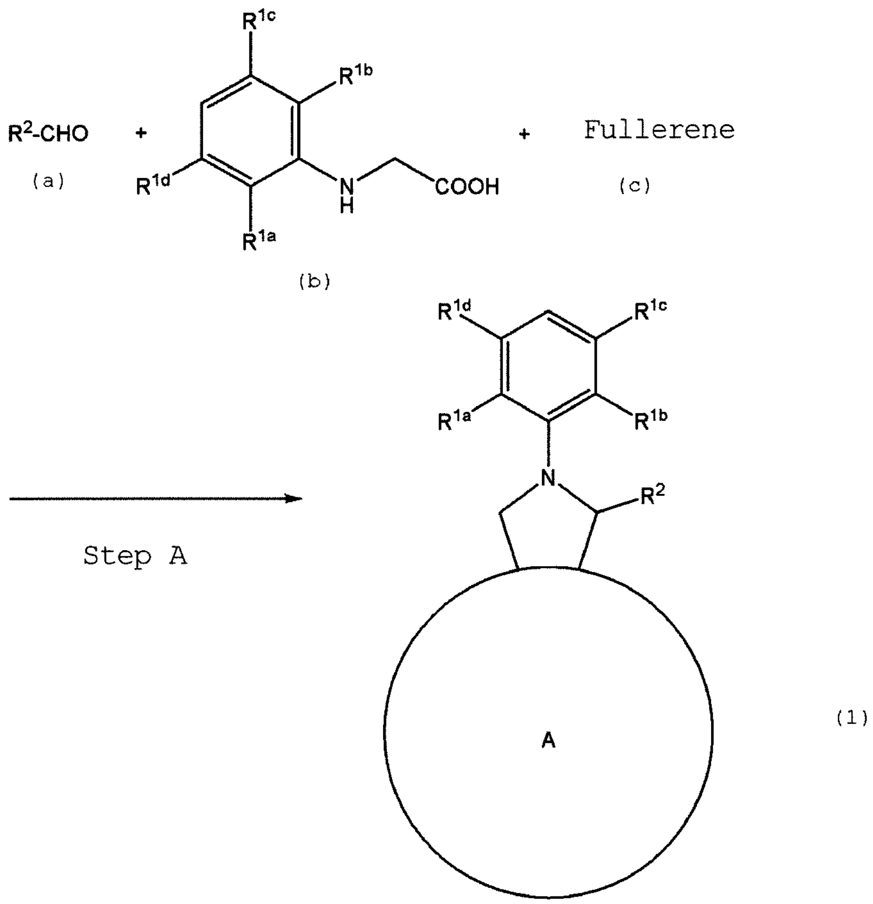


Step A

In step A, a glycine derivative (compound (b)) reacts with an aldehyde compound (compound (a)) and a fullerene (compound (c)) to thereby obtain a fullerene derivative (compound (1)) represented by formula (1).

Although the amount ratio of the aldehyde compound (compound (a)), the glycine derivative (compound (b)), and the fullerene (compound (c)) is arbitrarily determined, the aldehyde compound (compound (a)) and the glycine derivative (compound (b)) are each typically added in an amount of 0.1 to 10 moles, and preferably 0.5 to 2 moles, per mole of the fullerene (compound (c)), from the standpoint of achieving high yield.

The reaction is carried out without a solvent or in a solvent. Examples of solvents include carbon disulfide, chloroform, dichloroethane, toluene, xylene, chlorobenzene, and dichlorobenzene. Of these, chloroform, toluene, chlorobenzene, and the like are preferable. These solvents may be mixed in suitable proportions.

The reaction temperature is typically within the range of room temperature to about 150°C, and preferably

within the range of about 80 to about 120°C. As used herein, the room temperature is within the range of 15 to 30°C.

The reaction time is typically within the range of about 1 hour to about 4 days, and preferably within the range of about 10 to about 24 hours.

The obtained compound (1) can optionally be purified by a conventional purification method. For example, the obtained compound (1) can be purified by silica gel column chromatography (as a developing solvent, for example, hexane-chloroform, hexane-toluene, or hexane-carbon disulfide is preferably used), and further purified by HPLC (preparative GPC) (as a developing solvent, for example, chloroform or toluene is preferably used).

The aldehyde compound (compound (a)), the glycine derivative (compound (b)), and the fullerene (compound (c)) used in step A are all known compounds; the compounds can be synthesized by a known method or a method complying with a known method, and are also commercially available.

Specifically, the aldehyde compound (compound (a)) can be synthesized, for example, by the below-described method (a1), (a2), or (a3).

In the reaction formulae describing these methods, $R^2$ is as defined in formula (1), and corresponds to $R^2$ of the desired fullerene derivative.

Method (a1): Oxidation of Alcohol Represented by $R^2$-$CH_2OH$

For oxidation in this method, for example, the following known methods can be used: (i) a method using chromic acid, manganese oxide, or the like as an oxidant, (ii) swern oxidation using dimethylsulfoxide as an oxidant, or (iii) an oxidation method using hydrogen peroxide, oxygen, air, or the like in the presence of a catalyst.

Method (a2): Reduction of Carboxylic Acid Represented by $R^2$-COOH, Acid Halide Thereof, Ester Thereof, or Acid Amide Thereof

For reduction in this method, for example, the following known methods can be used: (i) a method using metal hydride as a reducing agent, (ii) a method comprising hydrogen reduction in the presence of a catalyst, or (iii) a method using hydrazine as a reducing agent.

Method (a3): Carbonylation of Halide Represented by $R^2$-X (X represents a halogen)

For carbonylation in this method, for example, a method comprising forming an anion from the halide using n-BuLi and introducing a carbonyl group thereinto can be used. As a carbonyl group-introducing reagent, amide compounds such as *N,N*-dimethylformamide (DMF); or N-formyl derivatives of piperidine, morpholine, piperazine, or pyrrolidine can be used.

Specifically, the glycine derivative (compound (b)) can be synthesized, for example, by the below-described method (b1), (b2), or (b3).

In the reaction formulae showing these methods, $Ar^1$ is a group represented by the following formula:

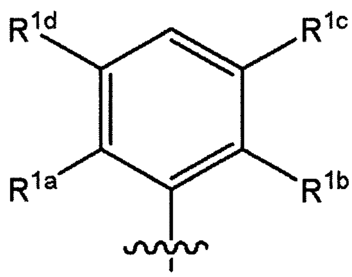

wherein the symbols are as defined above.

Method (b1): Reaction between Aniline Derivative and Halogenated Acetic Acid

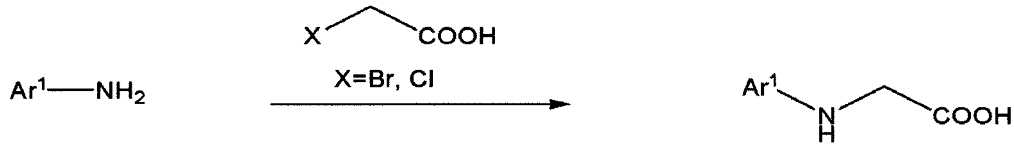


The reaction can employs water, methanol, ethanol, or a mixture thereof as a solvent, and can optionally be carried out as necessary in the presence of a base.

Method (b2): Reaction between Aniline Derivative and Halogenated Acetic Acid Ester, and Hydrolysis of Glycine Derivative Ester Obtained by Reaction

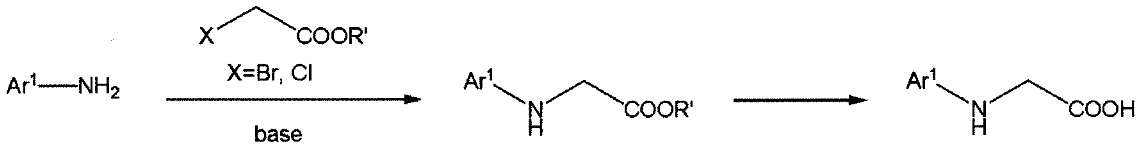


In this method, the reaction between an aniline derivative and a halogenated acetic acid ester can employs, for example, methanol or ethanol as a solvent, and can be carried out in the presence of a base such as acetate, carbonate, phosphate, and a tertiary amine. The hydrolysis of a glycine derivative ester can typically be carried out in the presence of a watersoluble alkali at room temperature.

Method (b3): Reaction between Aromatic Halide and Glycine

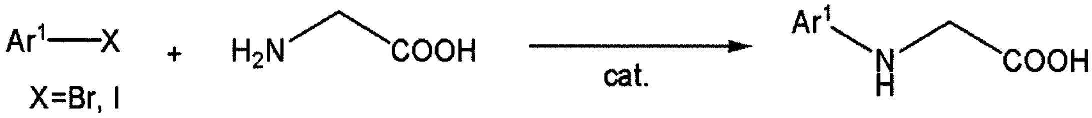


The reaction employs, for example, monovalent copper as a catalyst, and can be carried out in the presence of a bulky amine, an amino acid, or an amino alcohol. As a reaction solvent, water, methanol, ethanol, or a mixture thereof is preferably used. The reaction temperature is from room temperature to about 100°C.

As described above, the fullerene derivative according to the present invention can be synthesized by a simple method using a glycine derivative and an aldehyde derivative as starting materials; thus, the fullerene derivative can be produced at low cost.

Use of Fullerene Derivative

The fullerene derivative according to the present invention can be suitably used as an n-type semiconductor material, particularly as an n-type semiconductor material for photoelectric conversion elements such as organic thin-film solar cells.

When used as an n-type semiconductor material, the fullerene derivative according to the present invention is typically used in combination with an organic p-type semiconductor material (organic p-type semiconductor compound),

Examples of organic p-type semiconductor materials include poly-3-hexylthiophene (P3HT), poly-p-phenylenevinylene, poly-alkoxy-p-phenylenevinylene, poly-9,9-dialkylfluorene, and poly-p-phenylenevinylene.

Because of the many approaches to use these materials in solar cells in the past and their ready availability, these materials can easily provide devices that exhibit stable performance.

To achieve higher conversion efficiency, donor-acceptor type $\pi$-conjugated polymers capable of absorbing long-wavelength light because of their narrowed bandgap (low bandgap) are effective.

These donor-acceptor $\pi$-conjugated polymers comprise donor units and acceptor units, which are alternately positioned.

Examples of usable donor units include benzodithiophene, dithienosilole, and *N*-alkyl carbazole, and examples of usable acceptor units include benzothiadiazole, thienothiophene, and thiophene pyrrole dione.

Specific examples include high-molecular compounds obtained by combining these units, such as poly(thieno[3,4-b]thiophene-co-benzo[1,2-b:4,5-b']thiophene) (PTBx series), and poly(dithieno[1,2-b:4,5-b'][3,2-b:2',3'-d]silole-alt-(2,1,3-benzothiadiazole).

Of these, the following are preferable:

(1) poly({4,8-bis[(2-ethylhexyl)oxy]benzo[1,2-b:4,5-b']dithiophene-2,6-diyl}{3-fluoro-2-[(2-ethylhexyl)carbonyl]thieno[3,4-b]thiophenediyl}) (PTB7, the structural formula is shown below);
(2) poly[(4,8-di(2-ethylhexyloxy)benzo[1,2-b:4,5-b']dithiophene)-2,6-diyl-alt-((5-octylthieno[3,4-c]pyrrol-4,6-dione)-1,3-diyl) (PBDTTPD, the structural formula is shown below);
(3) poly[(4,4'-bis(2-ethylhexyl)dithieno[3,2-b:2',3'-d]silole)-2,6-diyl-alt-(2,1,3-benzothiadiazole)-4,7-diyl](PSBTBT, the structural formula is shown below);
(4) poly[N-9"-heptadecanyl-2,7-carbazole-alt-5,5-(4',7'-di-2-thienyl-2',1',3'-benzothiadiazole)](PCDTBT, the structural formula is shown below); and
(5) poly[1-(6-{4,8-bis[(2-ethylhexyl)oxy]-6-methylbenzo[1,2-b:4,5-b']dithiophene-2-yl}{3-fluoro-4-methylthieno[3,4-b]thiophene-2-yl}-1-octanone) (PBDTTT-CF, the structural formula is shown below)

Of these, more preferable examples include PTB-based compounds comprising as an acceptor unit thieno[3,4-b]thiophene having a fluorine atom at position 3, and yet more preferable examples include PBDTTT-CF and PTB7.

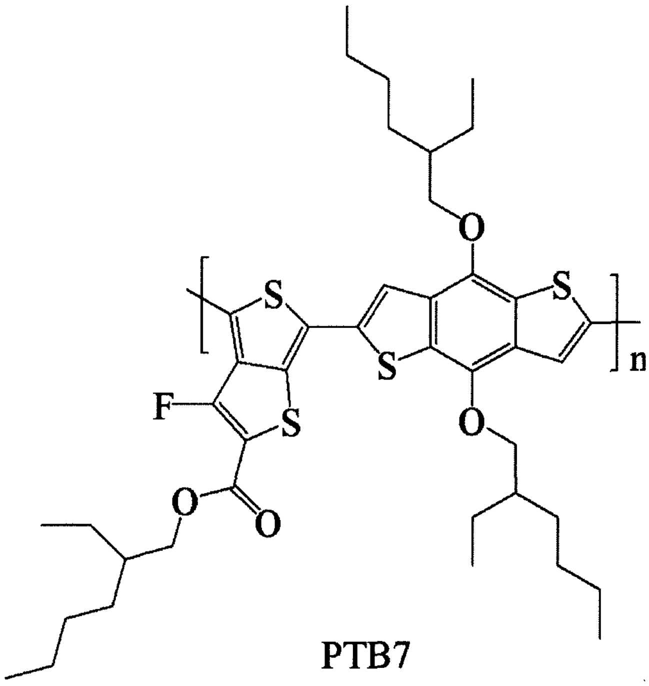

PTB7

wherein n represents the number of repeating units.

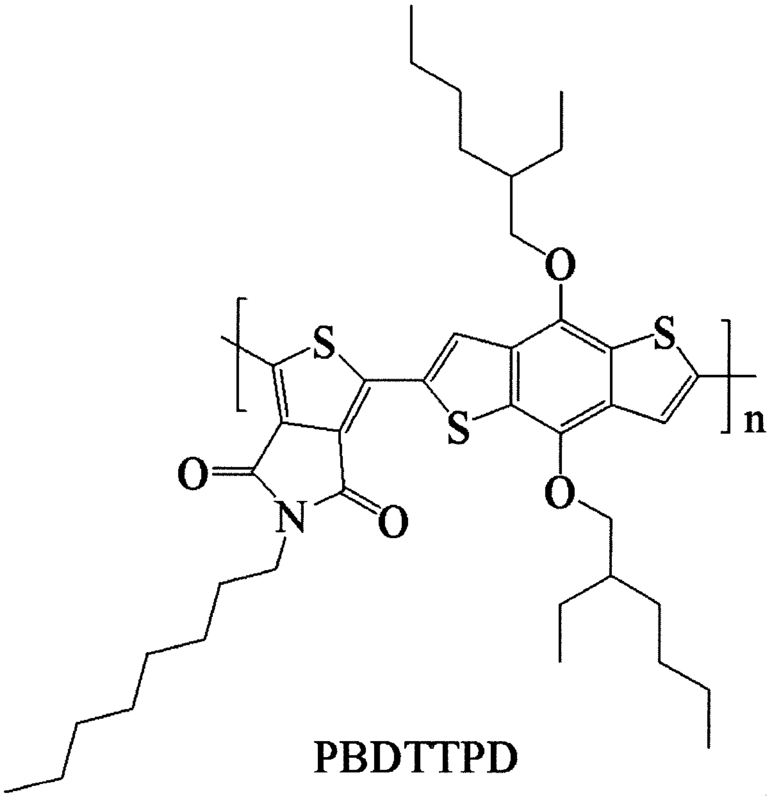

PBDTTPD

wherein n represents the number of repeating units.

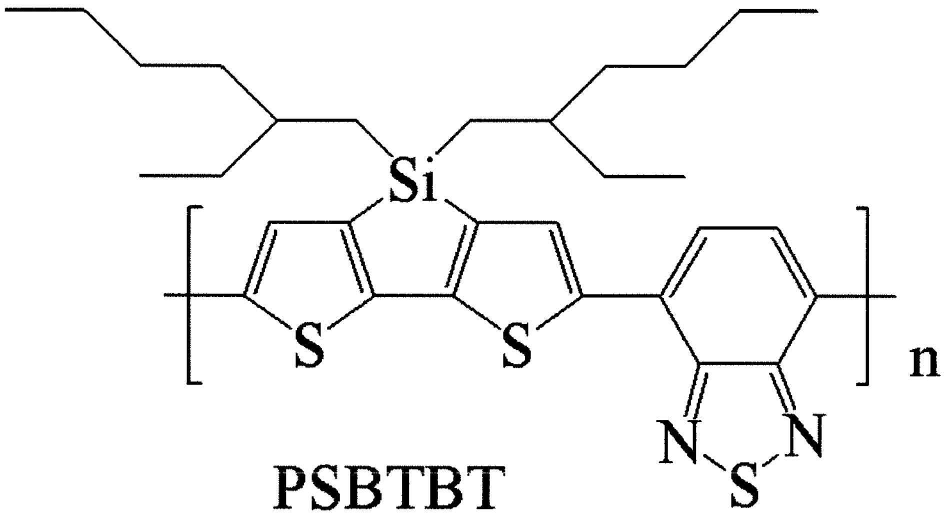

PSBTBT

wherein n represents the number of repeating units.

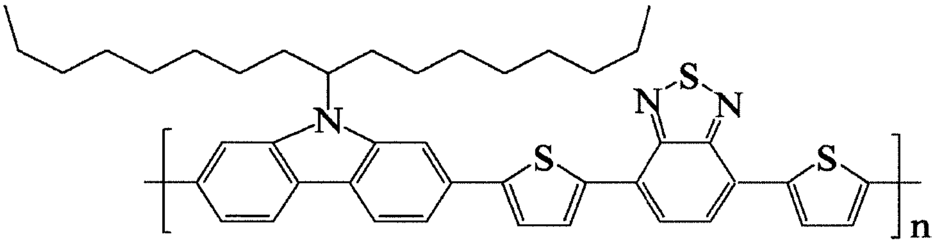

PCDTBT

wherein n represents the number of repeating units.

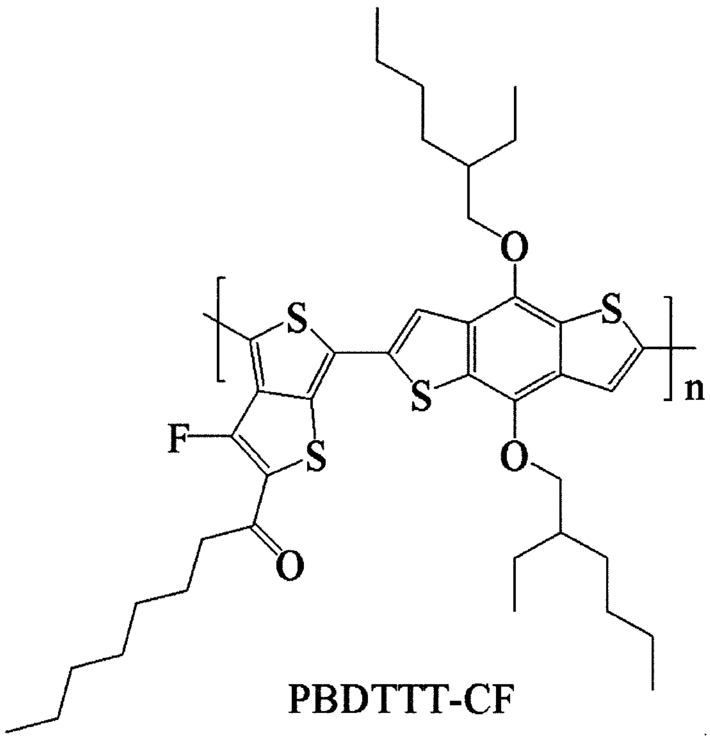

PBDTTT-CF

wherein n represents the number of repeating units.

An organic power-generating layer prepared by using the fullerene derivative according to the present invention as an n-type semiconductor material in combination with an organic p-type semiconductor material can achieve high conversion efficiency.

Because of its excellent solubility in various organic solvents, the fullerene derivative according to the present invention, when used as an n-type semiconductor material, enables the preparation of an organic power-generating layer by a coating technique, and also simplifies the preparation of an organic power-generating layer having a large area.

The fullerene derivative according to the present invention is a compound having excellent compatibility with organic p-type semiconductor materials as well as a suitable self-aggregating property. Thus, the fullerene derivative, when used as an n-type semiconductor material (organic n-type semiconductor material), can easily form an organic power-generating layer having a bulk junction structure. The use of such an organic power-generating layer enables the production of an organic thin-film solar cell or photosensor with high conversion efficiency.

Accordingly, the use of the fullerene derivative according to the present invention as an n-type semiconductor material enables the production of an organic thin-film solar cell having excellent performance at low cost.

An alternative application of the organic power-generating layer comprising (or consisting of) the n-type semiconductor material of the present invention is the use of the layer in an image sensor for digital cameras. In response to the demand for advanced functions (higher definition) in digital cameras, existing image sensors consisting of a silicon semiconductor are considered to suffer from lower sensitivity. Amid the demand, recent years have seen promises of achieving higher sensitivity and higher definition by using an image sensor consisting of an organic material with high photosensitivity. Materials for forming the light-receiving part of such a sensor need to absorb light with a high sensitivity

and efficiently generate an electrical signal therefrom. In response to this demand, because of its ability to efficiently convert visible light into electrical energy, an organic power-generating layer comprising (or consisting of) the n-type semiconductor material of the present invention can have high performance as a material for the above-described light-receiving part of the sensor.

n-Type Semiconductor Material

The n-type semiconductor material according to the present invention consists of a fullerene derivative according to the present invention.

Organic Power-Generating Layer

The organic power-generating layer according to the present invention comprises a fullerene derivative of the present invention as an n-type semiconductor material (n-type semiconductor compound).
The organic power-generating layer according to the present invention can be a light conversion layer (photoelectric conversion layer).
The organic power-generating layer according to the present invention typically comprises the aforementioned organic p-type semiconductor material (organic p-type semiconductor compound) in combination with the fullerene derivative according to the present invention, i.e., the n-type semiconductor material according to the present invention.
The organic power-generating layer according to the present invention typically consists of the n-type semiconductor material according to the present invention and the organic p-type semiconductor material.
The organic power-generating layer according to the present invention preferably has a bulk heterojunction structure formed by the n-type semiconductor material of the present invention and the organic p-type semiconductor material.
The organic power-generating layer according to the present invention is prepared, for example, by dissolving the n-type semiconductor material of the present invention and the aforementioned organic p-type semiconductor material in an organic solvent, and forming a thin film from the obtained solution on a substrate using a known thin-film forming technique, such as spin coating, casting, dipping, inkjet, and screen printing.
In formation of thin-film of an organic power-generating layer, the fullerene derivative according to the present invention has excellent compatibility with organic p-type semiconductor materials (preferably, P3HT, or PTB7) and suitable self-aggregating property. Therefore, it enables easy production of an organic power-generating layer comprising the fullerene derivative of the present invention, as an n-type semiconductor material, and an organic p-type semiconductor material, with the layer formed in a bulk heterojunction structure.

Organic Thin-Film Solar Cell

The organic thin-film solar cell according to the present invention comprises the above-described organic power-generating layer of the present invention.
Thus, the organic thin-film solar cell of the present invention exhibits high conversion efficiency.
The structure of the organic thin-film solar cell is not particularly limited, and the organic thin-film solar cell may have the same structure as that of a known organic thin-film solar cell. The organic thin-film solar cell according to the present invention can also be produced in accordance with a known method for producing an organic thin-film solar cell.
One example of the organic thin-film solar cell comprising the fullerene derivative is a solar cell comprising, disposed on a substrate in series, a transparent electrode (negative electrode), a charge transport layer on the negative electrode side, an organic power-generating layer, a charge transport layer on the positive electrode side, and an opposite electrode (positive electrode). The organic power-generating layer is preferably a thin-film semiconductor layer (i.e., a photoelectric conversion layer) comprising an organic p-type semiconductor material and the fullerene derivative of the present invention as an n-type semiconductor material, with the layer formed in a bulk heterojunction structure.
In solar cells having the above-described structure, known materials can suitably be used as materials for layers other than the organic power-generating layer. Specific examples of electrode materials include aluminium, gold, silver, copper, and indium tin oxide (ITO). Examples of charge transport layer materials include PFN(poly[9,9-bis(3'-(N,N-dimethylamino)propyl-2,7-fluorene)-alt-2,7-(9,9-dioctylfluorene)]) and $MoO_3$ (molybdenum oxide).

Photosensor

As described above, the photoelectric conversion layer obtained by the present invention can effectively function as an image sensor light-receiving part of advanced digital cameras. As compared with conventional photosensors including a silicon photodiode, a photosensor including the photoelectric conversion layer obtained by the present invention can receive an image in a well-lighted area without overexposure as well as a clear image in a poorly lighted area. This makes it possible to obtain an image with higher quality than those of conventional cameras. An photosensor comprises a silicon substrate, an electrode, a light-receiving part consisting of a photoelectric conversion layer, a color filter, and a microlens. The light-receiving part can be about several hundred nanometers in thickness, a fraction of the thickness of conventional silicon photodiodes.

Examples

The following Examples describe the present invention in more detail. However, the present invention is not limited to the Examples.

The annotation of the symbols and abbreviations used in the Examples is shown below. In addition, symbols and abbreviations typically used in the technical field to which the present invention pertains may also be used throughout this specification.

s: singlet
d: doublet
d-d: double doublet
t: triplet
m: multiplet
Calcd: calculated value
Found: actual measured value

In the following Examples, GPC columns manufactured by Japan Analytical Industry Co., Ltd. were used (2 columns, 2H and 1H, of the Jaigel H Series were connected for use).

Synthesis Example 1: Synthesis of Compound 1

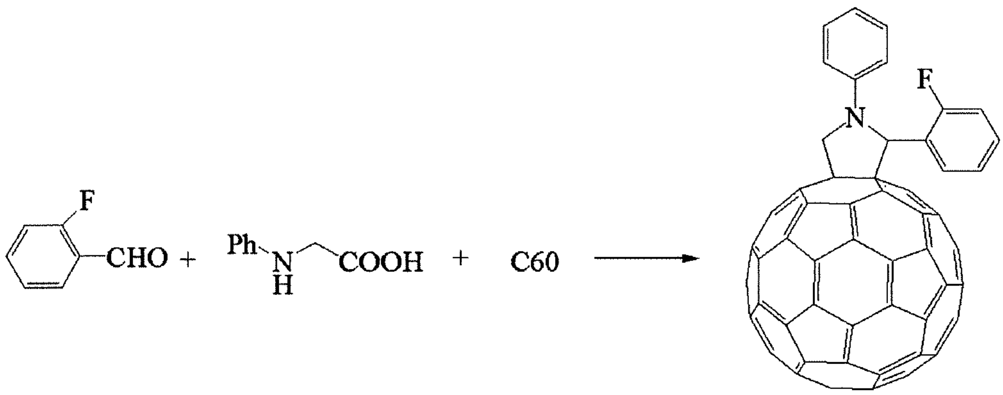

1

2-fluorobenzaldehyde (62 mg, 0.5 mmol), N-phenylglycine (151 mg, 1 mmol) and $C_{60}$ fullerene (350 mg, 0.5 mmol) were stirred in 100 mL of toluene at 120°C for 15 hours. After cooling, the solvent was distilled off, and the reaction product was separated by column chromatography ($SiO_2$, n-hexane:toluene = 20:1 to 5:1) to obtain Compound 1 (72.1 mg, yield: 15.4%). Compound 1 was further purified by preparative GPC (chloroform). $^1$H-NMR ($CDCl_3$) δ: 5.09 (1H, d, J = 9.9 Hz), 5.65 (1H, d, J = 9.9 Hz), 6.61 (1H, s), 7.02 - 7.18 (3H, m), 7.20 - 7.28(2H, m), 7.28 - 7.42 (4H, m), 7.84 (1H, d-d, J = 6.3, 6.3 Hz).
$^{19}$F-NMR ($CDCl_3$)δ: -114.0 - -115.5 (m).
MS (FAB) m/z 934 (M+1). HRMS calcd for $C_{74}H_{13}FN$ 934.1032; found 934.1023.

Synthesis Example 2: Synthesis of Compound 2

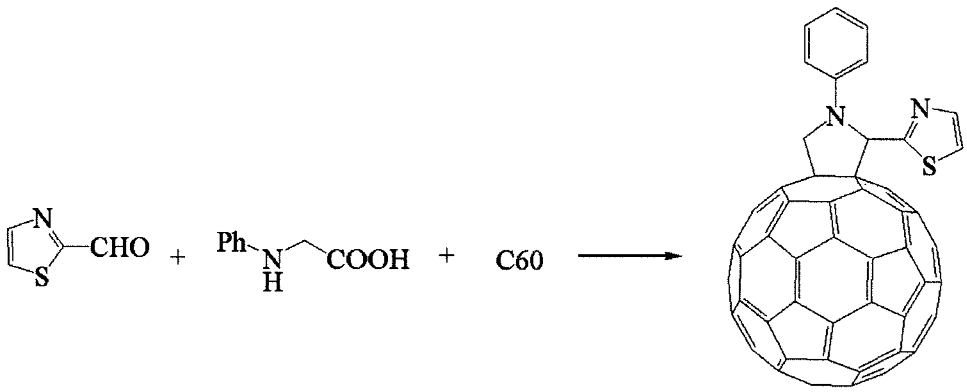

2

2-thiazole carbaldehyde (56 mg, 0.5 mmol), N-phenylglycine (76 mg, 0.5 mmol), and $C_{60}$ fullerene (175 mg, 0.25 mmol) were stirred in 100 mL of toluene at 120°C for 62 hours. After cooling, the solvent was distilled off, and the reaction product was seprated by column chromatography ($SiO_2$, n-hexane:toluene = 1:1 to toluene) to obtain Compound 2 (95 mg, yield: 41%). Compound 2 was further purified by preparative GPC (chloroform).
$^1$H-NMR ($CDCl_3$) δ: 5.27 (1H, d, J = 9.9 Hz), 5.78 (1H, d, J = 9.9 Hz), 6.91 (1H, s), 7.06 (1H, t, J = 7.1 Hz), 7.30 - 7.46 (5H, m), 7.84 (1H, D, J = 3.2 Hz).
MS (FAB) m/z 922 (M+) . HRMS calcd for $C_{71}H_{10}N_2S$ 922.0565; found 922.0562.

Synthesis Example 3: Synthesis of Compound 3

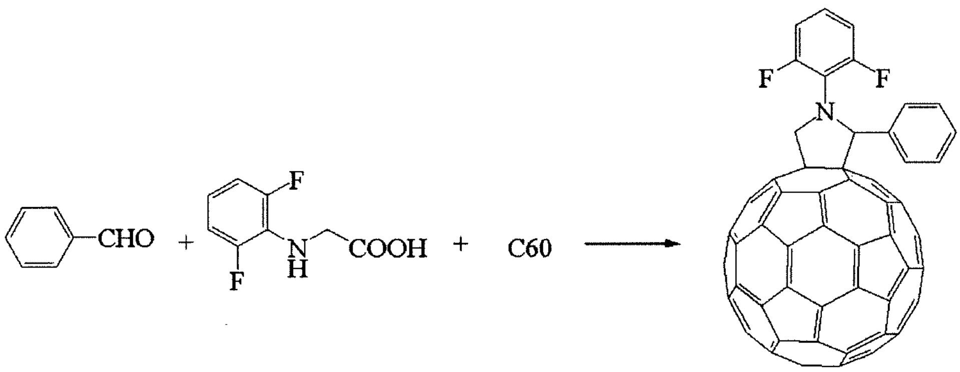

3

$C_{60}$ fullerene (360 mg, 0.5 mmol), benzaldehyde (212 mg, 2 mmol), and *N*-(2,6-difluorophenyl)glycine (187 mg, 1 mmol) were stirred in chlorobenzene (100 mL) at 130°C for 4 days. After cooling, the solvent was distilled off, and the reaction product was separated by silica gel column chromatography (n-hexane:toluene = 20:1 to 5:1) to obtain Compound 3 (108 mg, yield: 22.8%). Compound 3 was further purified by preparative GPC (chloroform).
$^1$H-NMR ($CDCl_3$) δ: 5.12 (1H, d, J = 9.1 Hz), 5.26 (1H, d, J = 9.1 Hz), 6.46 (1H, s), 6.96 (2H, t, J = 8.7 Hz), 7.12 - 7.35 (4H, m), 7. 77 (2H, d, J = 7.5 Hz).
$^{19}$F-NMR ($CDCl_3$)δ: -117.06 - -117.15 (m).
MS (FAB) m/z 951 (M+). HRMS calcd for $C_{74}H_{11}F_2N$ 951.0860; found 951.0861.

Synthesis Example 4: Synthesis of Compound 4

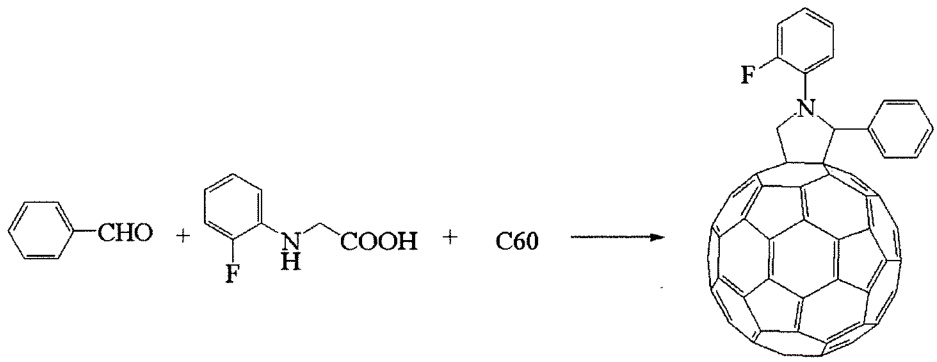

4

Fullerene $C_{60}$ (360 mg, 0.5 mmol), benzaldehyde (106 mg, 1 mmol), and *N*-(2-fluorophenyl)glycine (169 mg, 1 mmol) were stirred in chlorobenzene (100 mL) at 130°C for 4 days. After cooling, the solvent was distilled off, and the reaction product was separated by silica gel column chromatography (n-hexane:toluene = 20:1 to 5:1) to obtain Compound 4 (177 mg, yield: 37.9%). Compound 4 was further purified by preparative GPC (chloroform).
$^1$H-NMR($CDCl_3$) $\delta$:4.74 (1H, d, J = 9.6 Hz), 5.66 (1H, d, J = 9.6 Hz), 6.10 (1H, s), 7.10 -7.38 (7H, m), 7.77 (2H, d, J = 7.3 Hz). $^{19}$F-NMR($CDCl_3$) $\delta$: -119. 50 -119.75 (m).
MS (FAB) m/z 934 (M+1). HRMS calcd for $C_{74}H_{13}FN$ 934.1032; found 934.1052.

Synthesis Example 5: Synthesis of Compound 5

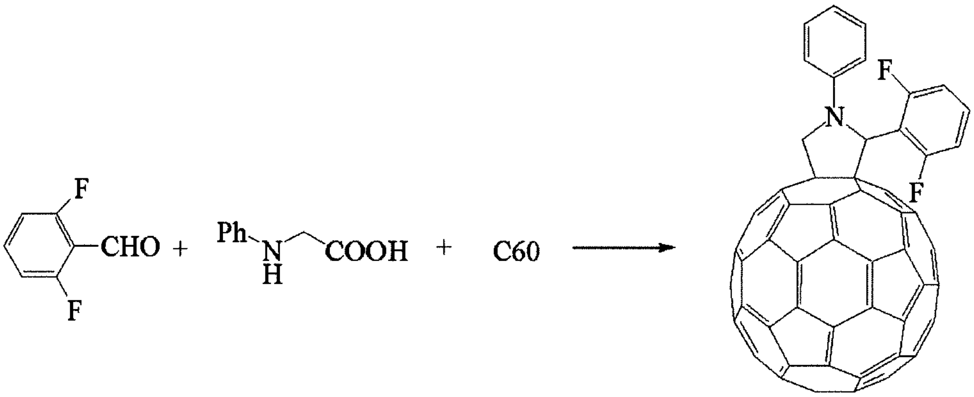

5

2,6-difluorobenzaldehyde (36 mg, 0.25 mmol), N-phenylglycine (76 mg, 0.5 mmol), and $C_{60}$ fullerene (175 mg, 0.25 mmol) were stirred in 100 mL of toluene at 120°C for 48 hours. After cooling, the solvent was distilled off, and the reaction product was separated by silica gel column chromatography ($SiO_2$, n-hexane:toluene = 20:1) to obtain Compound 5 (103 mg, yield: 43%). Compound 5 was further purified by preparative GPC (chloroform).
$^1$H-NMR($CDCl_3$)$\delta$: 5.40 (1H, d-d, J = 9.9, 5.9 Hz), 5.66 (1H, d-d, J = 9.9, 2.4 Hz), 6.88 - 7.02 (2H, m), 7.14 (1H, d, J = 7.5 Hz), 7.20 - 7.30 (3H, m), 7.37 (1H, t, J = 7.5 Hz).
$^{19}$F-NMR($CDCl_3$)$\delta$:-105.30 - -105.39 (1F), -114.35 - -114.45 (1F). MS (FAB) m/z 951 (M+) . HRMS calcd for $C_{74}H_{11}F_2N$ 951.0860; found 951.0867.

Synthesis Example 6: Synthesis of Compound 6

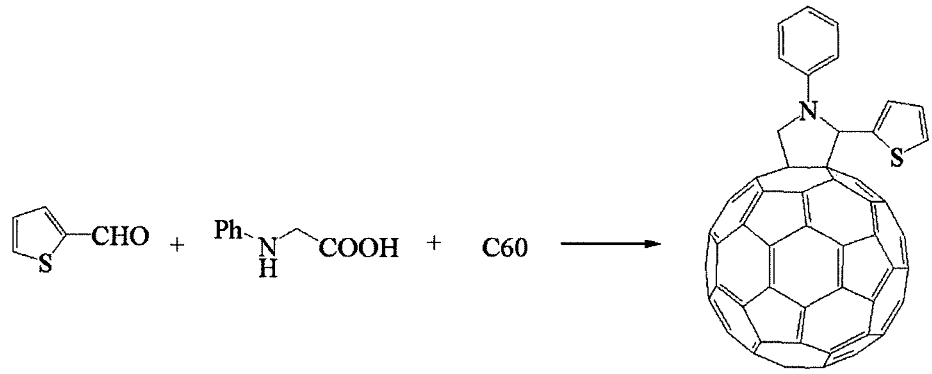

6

2-thiophenecarbaldehyde (56 mg, 0.5 mmol), *N*-phenylglycine (76 mg, 0.5 mmol), and $C_{60}$ fullerene (175 mg, 0.25 mmol) were stirred in 100 mL of toluene at 120°C for 60 hours. After cooling, the solvent was distilled off, and the reaction product was separated by column chromatography ($SiO_2$, n-hexane:toluene = 10:1 to 2:1) to obtain Compound 6 (113 mg, yield: 49%). Compound 6 was further purified by preparative GPC (chloroform).
$^1$H-NMR ($CDCl_3$) δ: 5.11 (1H, d, J = 10.1 Hz), 5.60 (1H, d, J = 10.1 Hz), 6.60 (1H, s), 6.96 - 7.02(1H, m), 7.04 - 7.12(1H, m), 7.22-7.30 (1H, m) 7.32 - 7.48(5H, m).

Synthesis Example 7: Synthesis of Compound 8

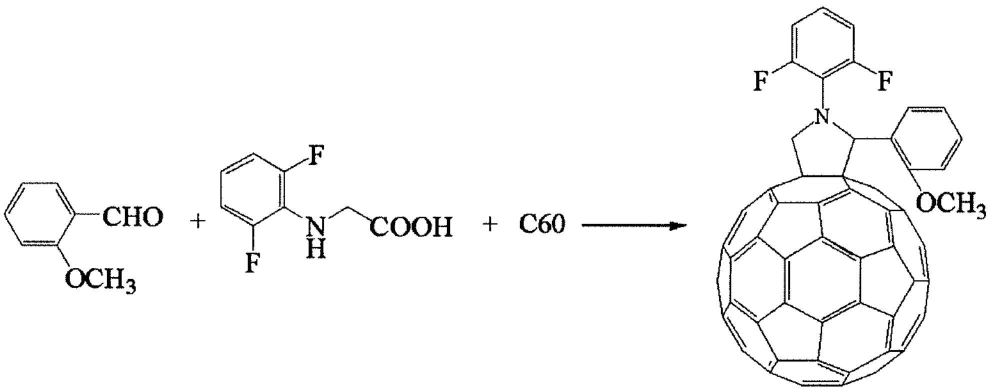

7

$C_{60}$ fullerene (360 mg, 0.5 mmol), 2-anisaldehyde (136 mg, 1 mmol), and *N*-(2,6-difluorophenyl)glycine (187 mg, 1 mmol) were stirred in chlorobenzene (100 mL) at 140°C for 4 days. After cooling, the solvent was distilled off, and the reaction product was separated by silica gel column chromatography (n-hexane:toluene = 10:1 to 5:1) to obtain Compound 7 (219 mg, yield: 44.6%). Compound 7 was further purified by preparative GPC (chloroform).
$^1$H-NMR($CDCl_3$)δ:3.86 (3H, s), 5.20 (1H, d, J = 9.1 Hz), 5.34 (1H, d, J = 9.1 Hz), 6.88 - 6.95 (2H, m), 6.99 - 7.07 (2H, m), 7.16 (1H, s), 7.20 - 7.29 (2H, m), 7.81 (1H, d, J = 7.9 Hz). $^{19}$F-NMR($CDCl_3$)δ:-114.80 - -114.98 (m).
MS (FAB) m/z 981 (M+) . HRMS calcd for $C_{75}H_{13}F_2NO$ 981.0965; found 981.0972.

Synthesis Example 8: Synthesis of Compound 8

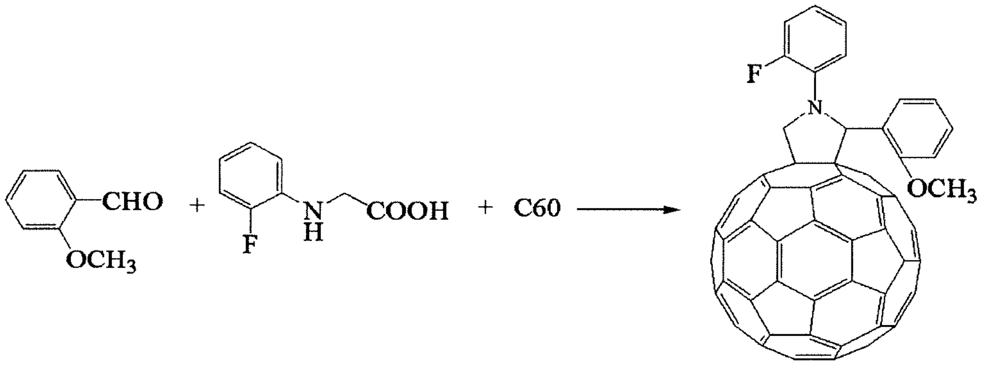

8

$C_{60}$ fullerene (180 mg, 0.25 mmol), 2-anisaldehyde (68 mg, 0.5 mmol), and N-(2-fluorophenyl)glycine (85 mg, 0.5 mmol) were stirred in chlorobenzene (80 mL) at 130°C for 4 days. After cooling, the solvent was distilled off, and the reaction product was separated by silica gel column chromatography (n-hexane:toluene = 20:1 to 2:1), followed by further purification by preparative GPC (chloroform) to thereby obtain Compound 8 (82.5 mg, yield: 34.2%).
$^{1}$H-NMR($CDCl_3$)δ :3.75 (3H, s), 4.71 (1H, d, J = 10.3 Hz), 5.65 (1H, d, J = 10.3 Hz), 6.68 (1H, s), 6.82 - 6.93 (2H, m), 7.02 - 7.29 (5H, m), 7.77 (1H, d-d, J = 7.9 , 1.6 Hz).
$^{19}$F-NMR ($CDCl_3$) δ: -121.23 - -121.34 (m).
MS (FAB) m/z 964 (M+1). HRMS calcd for $C_{75}H_{15}FNO$ 963.1059; found 963.1036.

Synthesis Example 9: Synthesis of Compound 9

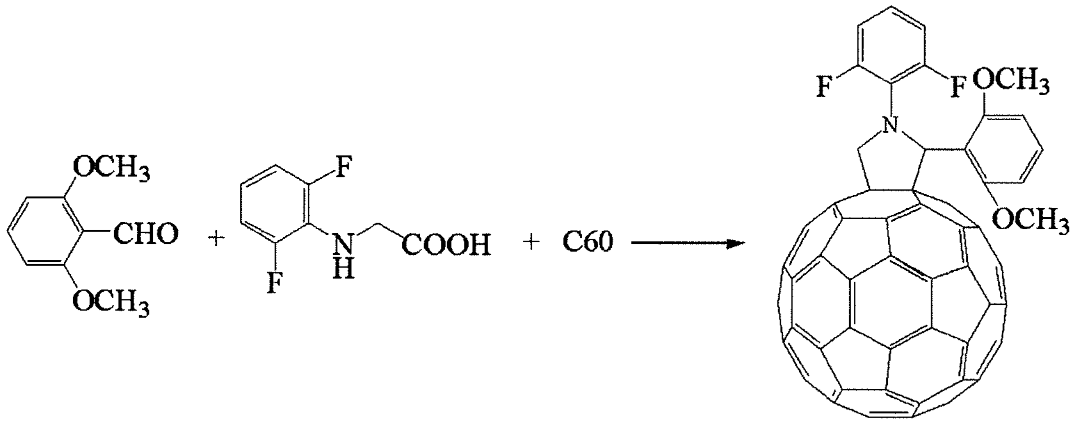

9

$C_{60}$ fullerene (360 mg, 0.5 mmol), 2,6-dimethoxybenzaldehyde (166 mg, 1 mmol), and N-(2,6-difluorophenyl)glycine (187 mg, 1 mmol) were stirred in chlorobenzene (100 mL) at 140°C for 2 days. After cooling, the solvent was distilled off, and the reaction product was separated by silica gel column chromatography (n-hexane:toluene = 10:1 to 5:1) to obtain Compound 9 (205 mg, yield: 40.5%). Compound 9 was further purified by preparative GPC (chloroform).
$^{1}$H-NMR($CDCl_3$)δ:3.66 (3H, s), 3.78 (3H, s), 5.04 (1H, d, J = 9.1 Hz), 5.52 (1H, d, J = 9.1 Hz), 6.38 (1H, d, J = 7.7 Hz), 6.55 (1H, d, J = 7.7 Hz), 6.86 - 6.95 (1H, m), 6.97 - 7.06 (1H, m), 7.11 - 7.20 (2H, m), 7.38 (1H, S).
$^{19}$F-NMR ($CDCl_3$) δ: -119.23 -118.31 (2F, m).
MS (FAB) m/z 1012 (M+1). HRMS calcd for $C_{76}H_{16}F_2NO_2$ 1012.1149;
found 1012.1116.

Synthesis Example 10: Synthesis of Compound 10

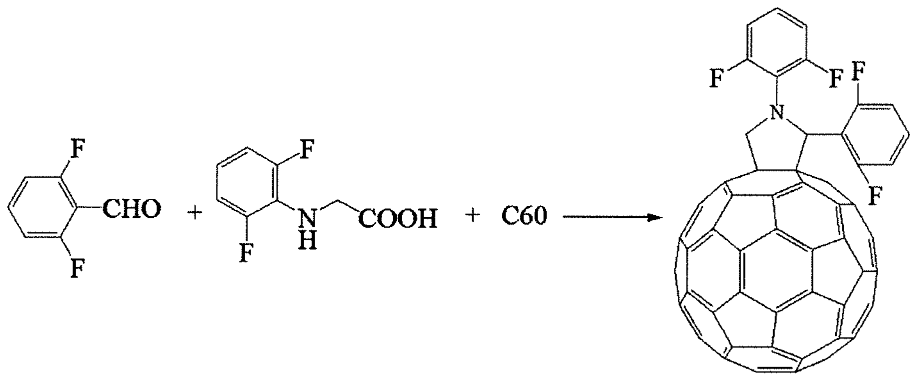

10

$C_{60}$ fullerene (360 mg, 0.5 mmol), 2,6-difluorobenzaldehyde (284 mg, 2 mmol), and N-(2,6-difluorophenyl)glycine (187 mg, 1 mmol) were stirred in chlorobenzene (100 mL) at 140°C for four days. After cooling, the solvent was distilled off, and the reaction product was separated by silica gel column chromatography (n-hexane:toluene = 20:1 to 5:1), followed by further purification by preparative GPC (chloroform) to thereby obtain Compound 10 (108.9 mg, yield: 22.0%).

$^1$H-NMR($CDCl_3$) δ:5.02 (1H, d-d, J = 9.6, 2.4 Hz), 5.41 (1H, d, J =9.6 Hz), 6.76 (2H, t, J = 8.8 Hz), 6.91 - 7.05 (3H, m), 7.06 (1H, d, J = 2.4 Hz), 7.08 - 7.18 (1H, m), 7.18 - 7.27 (1H, m). $^{19}$F-NMR($CDCl_3$) δ: -104.08 -104.17 (1F, m), -112.32 (1F, t, J = 7.9 Hz), -118.64 -118.76 (2F, m).

MS (FAB) m/z 988 (M+1). HRMS calcd for $C_{74}H_{10}F_4N$ 988.0749; found 988.0747.

Synthesis Example 11: Synthesis of Compound 11

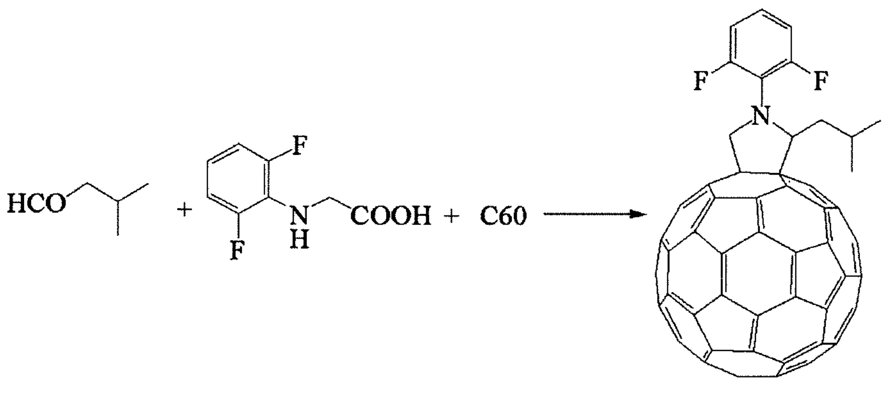

11

Fullerene $C_{60}$ (90 mg, 0.12 mmol), isovaleraldehyde (22 mg, 0.25 mmol), and *N*-(2,6-difluorophenyl)glycine (23 mg, 0.12 mmol) were stirred in chlorobenzene (60 mL) at 130°C for 3 days. After cooling, the solvent was distilled off, and the reaction product was separated by silica gel column chromatography (n-hexane:toluene = 20:1), followed by further purification by preparative GPC (chloroform) to thereby obtain Compound 11 (10.5 mg, yield: 9.0%).

$^1$H-NMR($CDCl_3$-$CS_2$) δ:0.95 (3H, d, J = 6.4 Hz), 1.03 (3H, d, J = 6.4Hz), 1.88 - 2.00 (1H, m), 2.36 - 2.46 (2H, m), 5.12 (1H, d, J = 9.6 Hz), 5.19 (1H, d, J = 9.6 Hz), 5.40 (1H, d-d, J = 6.4, 6.4 Hz), 7.00 - 7.35 (3H, m).

$^{19}$F-NMR ($CDCl_3$) δ: -117.13 - -117.20 (m).

MS (FAB) m/z 932 (M+1). HRMS calcd for $C_{72}H_{14}F_2N$ 931.1173; found 931.1206.

Synthesis Example 12: Synthesis of Compound 12

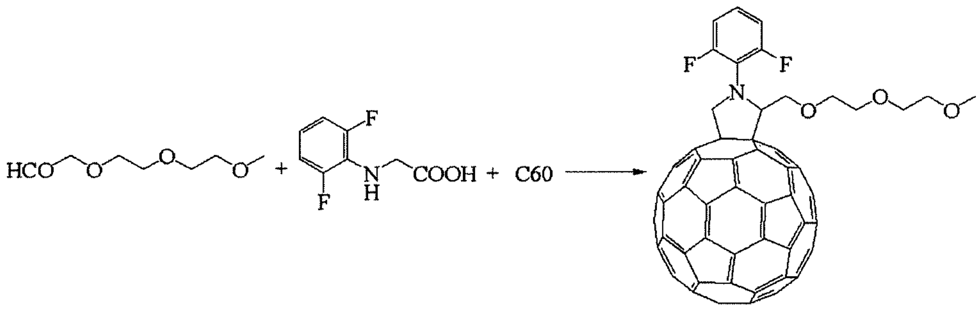

1 2

$C_{60}$ fullerene (180 mg, 0.25 mmol), 2,5,8-trioxadecanal (162 mg, 0.25 mmol), and *N*-(2,6-difluorophenyl)glycine (46.8 mg, 0.25 mmol) were stirred in chlorobenzene (80 mL) at 135°C for 4 days. After cooling, the solvent was distilled off, and the reaction product was separated by silica gel column chromatography (toluene:ethyl acetate = 50:1), followed by further purification by preparative GPC (chloroform) to thereby obtain Compound 12 (47.9 mg, yield: 19.0%).
$^1$H-NMR ($CDCl_3$) δ:3.33 (3H, s), 3.41 - 3.72 (6H, m), 4.28 (1H, d-d-d, J = 9.9, 5.5, 5.5 Hz), 4.47 (1H, d-d-d, J = 9.9, 5.5, 5.5 Hz), 5.12 (1H, d, J = 9.6 Hz), 5.19 - 5.26 (2H, m), 5.30 (1H, d, J = 9.6 Hz), 5.54 (1H, d-d, J = 6.0, 6.0 Hz), 7.06 (2H, d-d, J = 8.5, 8.5 Hz), 7.12 - 7.25 (1H, m).
$^{19}$F-NMR ($CDCl_3$)δ: -117.56 - -117.65 (m).
MS (FAB) m/z 1009 (M+1). HRMS calcd for $C_{74}H_{19}F_2NO_3$ 1007.1330; found 1007.1308.

Synthesis Example 13

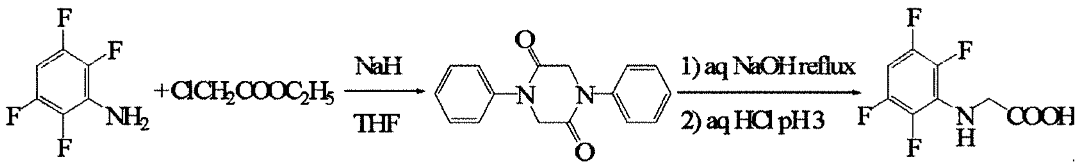


(1) Synthesis of *N*-(2,3,5,6-tetrafluorophenyl)glycine

The synthesis was carried out in the manner disclosed in a known document (Brooke, G.M. et al., Tetrahedron, 1971, vol. 27, page 5,653).

A solution of 2,3,5,6-tetrafluoroaniline (2.5 g, 15 mmol) in THF (25 mL) was added dropwise to a solution of sodium hydride (15 mmol) in THF (12 mL) over 1 hour at -30°C. After dropwise addition, the reaction mixture was stirred at room temperature for 1 hour. To this reaction mixture, a solution of ethyl chloroacetate (15 mmol) in THF (12 mL) was added dropwise at room temperature, followed by stirring while heating under reflux for 1 hour. After cooling, the reaction mixture was poured into ice water, and extracted with ether. After dehydration with magnesium sulfate, the extract was concentrated under reduced pressure (yield: 1.8 g). 0.3 g of this reaction product was stirred in 25 mL of a 30% aqueous sodium hydroxide solution under reflux for 3 hours. After cooling, the reaction mixture was adjusted to a pH of 3 with concentrated hydrochloric acid, and extracted with ethyl acetate. The organic phase was washed with water, dehydrated with magnesium sulfate, and concentrated under reduced pressure (yield: 1.2 g).
$^1$H-NMR($CD_3OD$)δ: 4.00 - 4.14 (2H, m), 6.48 - 6.61 (1H, m). $^{19}$F-NMR($CD_3OD$)δ: -144.11 - -144.25 (2F, m), -163.14 - -163.28 (2F, m).

(2) Synthesis of Compound 13

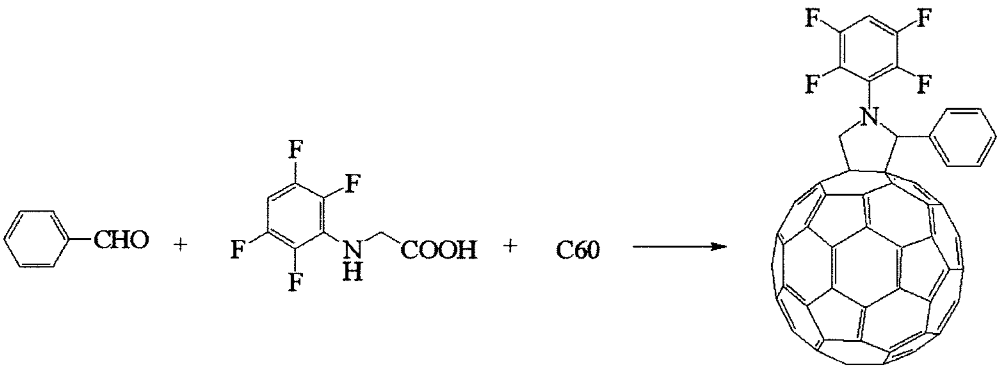

13

$C_{60}$ fullerene (180 mg, 0.25 mmol), benzaldehyde (1.06 g, 10 mmol), and *N*-(2,3,5,6-tetrafluorophenyl)glycine (45 mg, 0.2 mmol) were stirred in chlorobenzene (100 mL) at 145°C for 2 days. After cooling, the solvent was distilled off. The target product was confirmed by peaks at 4.60 (1H, d, J = 10.0 Hz), 5.66 (1H, D, J = 10.0 Hz), and 5.84 (1H, s) (δ, (ppm)) in $^1$H-NMR ($CDCl_3$), and peaks at -138.70 - -138.90 (2F, m), and -150.20 - -150.35 (2F, m) (δ (ppm)) in $^{19}$F-NMR ($CDCl_3$).

Compound 14: Synthesis of Compound 14

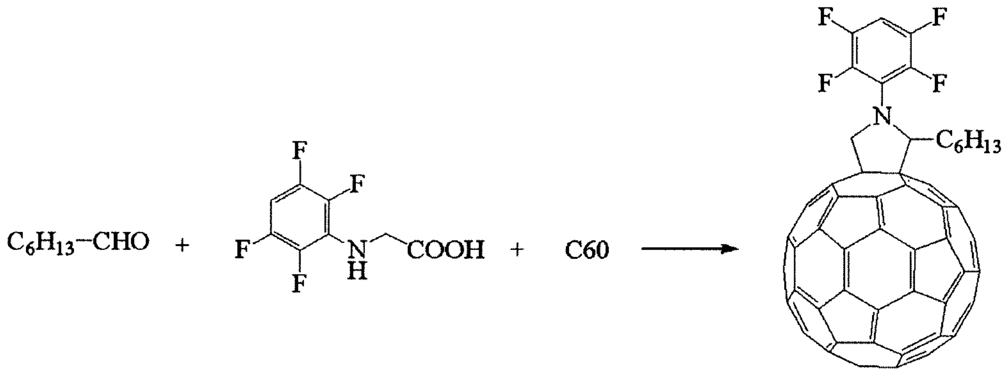

14

$C_{60}$ fullerene (180 mg, 0.25 mmol), pentanal (1 mL), and *N*-(2,3,5,6-tetrafluorophenyl)glycine (45 mg, 0.2 mmol) were stirred in chlorobenzene (100 mL) at 145°C for 4 days. After cooling, the solvent was distilled off, and the reaction product was separated by silica gel column chromatography (hexane:toluene = 20:1). The target product was obtained (11.8 mg, yield: 4.8%). $^1$H-NMR($CDCl_3$)δ:0.85 (3H, d, J = 6.4 Hz), 1.20 - 1.70 (8H, m), 2.40- 2.60 (2H, m), 5.11 (1H, d, J = 9.6Hz), 5.37 (1H, d, J = 9.6 Hz), 5.50 (1H, d-d, J = 6.4, 6.4 Hz), 6.85 - 7.00 (1H, m). $^{19}$F-NMR ($CDCl_3$) δ: -139.00 - -139.20 (2F, m), -146.60 - -146.80 (2F, m).

Synthesis of Compound 15

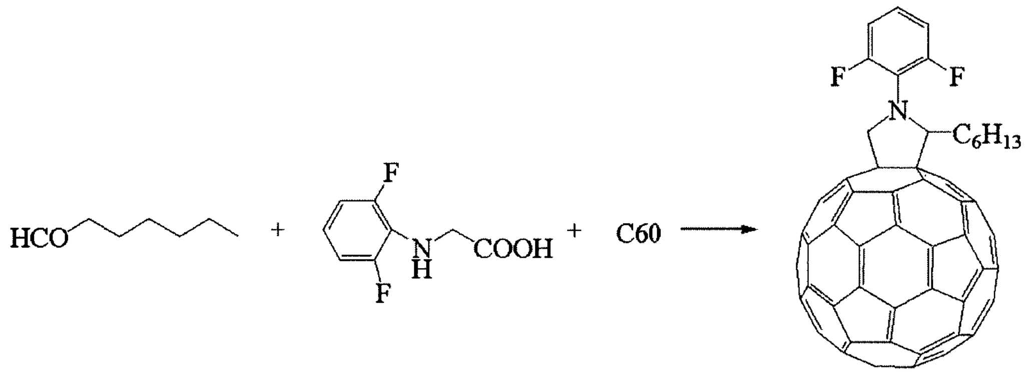

15

$C_{60}$ fullerene (360 mg, 0.50 mmol), pentanal (1 mL), and N-(2,6-difluorophenyl)glycine (94 mg, 0.50 mmol) were stirred in chlorobenzene (150 mL) at 130°C for 4 days. After cooling, the solvent was distilled off, and the reaction product was separated by silica gel column chromatography (n-hexane:toluene = 50:1), followed by further purification by preparative HPLC (column used: Cosmosil Buckyprep (20 ø × 250 mm); Nacalai Tesque, Inc.; solvent: toluene) to thereby obtain Compound 15 (35.5 mg, yield: 7.4%).

$^1$H-NMR($CDCl_3$) δ: 0.81 (3H, d, J = 6.9 Hz), 1.10 - 1.75 (8H, m), 2.25 - 2.36 (1H, m), 2.44 - 2.55 (1H, m), 5.08 (1H, d, J = 9.8 Hz), 5.18 (1H, d, J = 9.8 Hz), 5.50 (1H, d-d, J = 6.9, 5.9 Hz), 7.08 (2H, d-d, J = 8.7, 8.7 Hz), 7.15 - 7.28 (1H, m).

$^{19}$F-NMR ($CDCl_3$) δ: -117.76 (2F,d-d, J = 7.5, 7.0 Hz).

Test Example 1

Solar cell was produced in accordance with the following procedure using fullerene derivative obtained in Synthesis Example 4 as an n-type semiconductor material, and the performance was evaluated.

The following materials were used: PTB7 as an organic p-type semiconductor material, PFN(poly[9,9-bis(3'-(N,N-dimethylamino)propyl-2,7-fluorene)-alt-2,7-(9,9-dioctylfluorene)]) and $MoO_3$ (molybdenum oxide) as charge transport layer materials, and ITO (indium tin oxide) (negative electrode) and aluminium (positive electrode) as electrodes.

(1) Preparation of Solar Cell for Testing

Solar cells for testing were prepared in accordance with the following procedure.

1) Pretreatment on Substrate

An ITO patterning glass plate (manufactured by Sanyo Vacuum Industries Co.,Ltd.) was placed in a plasma cleaner (Harrick plasma, PDC-32G), and the surface of the substrate was washed with generated plasma while oxygen gas was being introduced for 10 minutes.

2) Preparation of PFN Thin Film (Charge Transport Layer on Negative Electrode Side)

A PFN thin film was formed using a PFN methanol solution (2% w/v) on the pretreated ITO glass plate by using an ABLE/ASS-301 spin-coating-film-forming apparatus. The formed PFN thin film had a thickness of about 10 nm.

3) Preparation of Organic Semiconductor Film (Organic Power-Generating Layer)

With the substrate placed in a glove box, the PFN thin film was spin-coated with a solution containing PTB7 which were dissolved in chlorobenzene beforehand and the fullerene derivative, and diiodooctane (3% v/v relative to chlorobenzene), using a MIKASA/MS-100 spin-coating film-forming apparatus at 1,000 rpm for 2 minutes to thereby obtain an organic semiconductor thin film (organic power-generating layer) of about 90 to 110 nm.

4) Vacuum Deposition of Charge Transport Layer on Positive Electrode Side and Vacuum Deposition of Metal Electrode

The above-prepared laminate was placed on a mask inside a compact high vacuum evaporator (Eiko Co., Ltd., VX-20). An $MoO_3$ layer (10 nm) as a charge transport layer on the positive electrode side and an aluminium layer (80 nm) as a metal electrode were deposited thereon in series using the high vacuum evaporator.

(2) Current Measurement by Pseudo Solar Light Irradiation

Current measurement using pseudo solar light irradiation was conducted by using SourceMeter (Keithley, Model 2400), current-voltage measuring software and a solar simulator (San-Ei Electric Co., Ltd., XES-301S).
The solar cells for testing produced in section (1) were irradiated with a given amount of pseudo solar light, and the generated current and voltage were measured. Energy conversion efficiency was then determined by the following equation.
Table 1 shows the measurement results of short-circuit current, open voltage, fill factor (FF), and conversion efficiency. The conversion efficiency is a value determined by the following equation.

$$\text{Conversion efficiency } \eta\ (\%) = FF\ (V_{oc} \times J_{sc}/P_{in}) \times 100$$

FF: Fill Factor, $V_{oc}$: Open voltage, $J_{sc}$: Short-circuit Current, Pin: Intensity of Incident Light (Density)

Table 1

| | Short-circuit Current (mA/cm$^2$) | Open Voltage (V) | FF | Conversion Efficiency (%) |
|---|---|---|---|---|
| Test Example 1 | 12.17 | 0.77 | 0.61 | 5.71 |

Test Example 2

Comparative Compound 1 of the below-described structural formula, and the fullerene derivatives obtained in Synthesis Examples 1 to 10 were used as n-type semiconductor materials to thereby prepare solar cells having the same cell structure as that of Test Example 1 in accordance with the following procedure. The performance of each fullerene derivative was then evaluated. Each of the fullerene derivatives was subjected to column separation, and further purified by HPLC (used column: Cosmosil Buckyprep (20 ø × 250 mm); Nacalai Tesque, Inc.; solvent:toluene) for use.

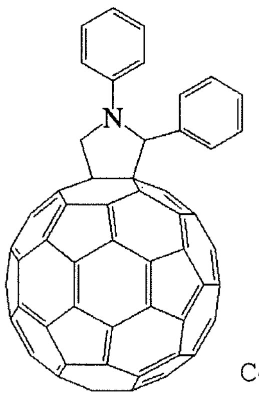

Comparative Compound 1

Table 2 shows the results.

Table 2

| Fullerene Derivative | p-type Semiconductor | Short-circuit Current (mA/cm$^2$) | Open Voltage (V) | FF | Conversion Efficiency (%) |
|---|---|---|---|---|---|
| Comparative Compound 1 | PTB7 | 14.54 | 0.74 | 0.66 | 7.16 |
| Compound 1 | PTB7 | 14.33 | 0.73 | 0.65 | 6.84 |
| Compound 3 | PTB7 | 14.84 | 0.78 | 0.61 | 7.09 |
| Compound 4 | PTB7 | 14.47 | 0.76 | 0.64 | 7.12 |
| Compound 5 | PTB7 | 14.85 | 0.76 | 0.61 | 6.88 |

(continued)

| Fullerene Derivative | p-type Semiconductor | Short-circuit Current ($mA/cm^2$) | Open Voltage (V) | FF | Conversion Efficiency (%) |
|---|---|---|---|---|---|
| Compound 7 | PTB7 | 14.76 | 0.80 | 0.55 | 6.45 |
| Compound 8 | PTB7 | 14.76 | 0.77 | 0.60 | 6.76 |
| Compound 9 | PTB7 | 13.88 | 0.81 | 0.55 | 6.10 |
| Compound 10 | PTB7 | 13.72 | 0.79 | 0.60 | 6.53 |
| Comparative Compound 2 | PTB7 | 14.21 | 0.76 | 0.67 | 7.27 |
| Compound 15 | PTB7 | 13.55 | 0.82 | 0.47 | 5.21 |

Test Example 3

Comparative compound 2 of the below-described structural formula and Compound 15 were used as n-type semiconductor materials to thereby produce solar cells having the same cell structures as those of Test Examples 1 and 2 in accordance with the following procedure. The performance of each fullerene derivative was then evaluated. Comparative Compounds 1 and 2 were synthesized in accordance with Patent Document 3.

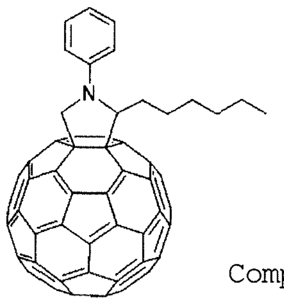

Comparative Compound 2

Compared with Comparative Examples 1 and 2 whose phenyl groups are not substituted (Comparative Compounds 1 and 2), the solar cells comprising the compounds according to the present invention all show improved open voltage. The open voltage also improves in accordance with the number of substituents.

This trend is more noticeable with the phenyl group attached to the pyrrolidine ring at position 1 (nitrogen) than the phenyl group attached to the pyrrolidine ring at position 2. Although there are some documents mentioning substituents of phenyl contained in a fullerene derivative and open voltage (below-listed documents 1) to 4)), there have been no such findings concerning phenyl attached to the nitrogen of a pyrrolidine-containing derivative. Further, there have been no previous examples of a fullerene derivative whose pyrrolidine ring is substituted with the above-described substituents at both positions 1 and 2.

Reference

1) Ito et al., Journal of Materials Chemistry, 2010, vol. 20, page 9,226 (Non-patent Document 1)
2) Hummelen et al., Organic Letters, 2007, vol. 9, page 551
3) Troshin et al., Advanced Functional Materials, 2009, vol. 19, page 779
4) JP2011-181719A


**Claims**

**1.** A fullerene derivative represented by formula (1):

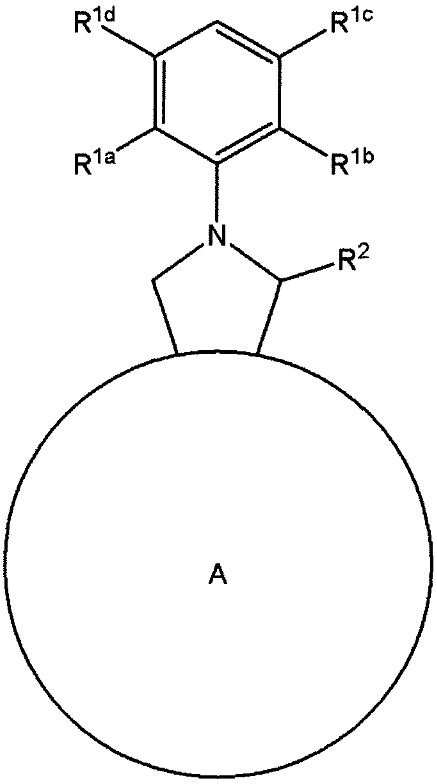

(1)

wherein

$R^{1a}$ and $R^{1b}$ are the same or different, and each represents a hydrogen atom or a fluorine atom;
$R^{1c}$ and $R^{1d}$ are the same or different, and each represents a hydrogen atom, a fluorine atom, alkyl optionally substituted with at least one fluorine atom, alkoxy optionally substituted with at least one fluorine atom, ester, or cyano;
$R^2$ represents

(1) phenyl optionally substituted with at least one substituent selected from the group consisting of fluorine, alkyl, alkoxy, ester, and cyano,
(2) a 5-membered heteroaryl group optionally substituted with 1 to 3 methyl groups, or
(3) alkyl, alkoxy, ether, acyl, ester, or cyano; and

ring A represents a fullerene ring;

with the proviso that when $R^{1a}$, $R^{1b}$, $R^{1c}$, and $R^{1d}$ are each a hydrogen atom, $R^2$ represents phenyl substituted with 1 or 2 fluorine atoms or a 5-membered heteroaryl group optionally substituted with 1 to 3 methyl groups.

**2.** The fullerene derivative according to Claim 1, wherein
$R^{1a}$ and $R^{1b}$ are the same or different, and each represents a hydrogen atom or a fluorine atom;
at least one of $R^{1a}$ and $R^{1b}$ is a fluorine atom; and
$R^2$ is a group represented by the following formula:

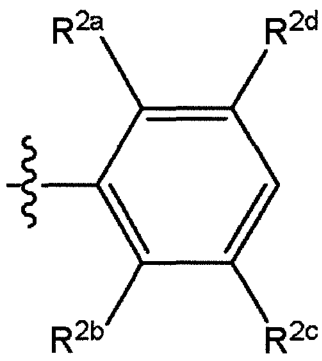

wherein,

$R^{2a}$ and $R^{2b}$ are the same or different, and each represents a hydrogen atom, a fluorine atom, alkyl, or alkoxy; and $R^{2c}$ and $R^{2d}$ are the same or different, and each represents a hydrogen atom, a fluorine atom, alkyl, alkoxy, ester, or cyano.

**3.** The fullerene derivative according to Claim 2, wherein
$R^{1a}$ and $R^{1b}$ are the same or different, and each represents a hydrogen atom or a fluorine atom;
at least one of $R^{1a}$ and $R^{1b}$ is a fluorine atom;
$R^{1c}$ and $R^{1d}$ are the same or different, and each represents a hydrogen atom or a fluorine atom;
$R^{2a}$ and $R^{2b}$ are the same or different, and each represents a hydrogen atom, a fluorine atom, alkyl, or alkoxy; and
$R^{2c}$ and $R^{2d}$ each represents a hydrogen atom.

**4.** The fullerene derivative according to any one of Claims 1 to 3, wherein the ring A is $C_{60}$ fullerene or $C_{70}$ fullerene.

**5.** An n-type semiconductor material consisting of the fullerene derivative according to any one of Claims 1 to 4.

**6.** The n-type semiconductor material according to Claim 5, which is for use in an organic thin-film solar cell.

**7.** An organic power-generating layer comprising the n-type semiconductor material according to Claim 6.

**8.** A photoelectric conversion element comprising the organic power-generating layer according to Claim 7.

**9.** The photoelectric conversion element according to Claim 8, which is an organic thin-film solar cell.

## INTERNATIONAL SEARCH REPORT

International application No.
PCT/JP2014/063127

A. CLASSIFICATION OF SUBJECT MATTER
*C07D209/70*(2006.01)i, *C07D409/04*(2006.01)i, *C07D417/04*(2006.01)i, *H01L51/05*(2006.01)i, *H01L51/30*(2006.01)i, *H01L51/46*(2006.01)i

According to International Patent Classification (IPC) or to both national classification and IPC

B. FIELDS SEARCHED

Minimum documentation searched (classification system followed by classification symbols)
C07D209/70, C07D409/04, C07D417/04, H01L51/05, H01L51/30, H01L51/46

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2014 |
| Kokai Jitsuyo Shinan Koho | 1971-2014 | Toroku Jitsuyo Shinan Koho | 1994-2014 |

Electronic data base consulted during the international search (name of data base and, where practicable, search terms used)
CAplus/REGISTRY(STN)

C. DOCUMENTS CONSIDERED TO BE RELEVANT

| Category* | Citation of document, with indication, where appropriate, of the relevant passages | Relevant to claim No. |
|---|---|---|
| A | JP 2012-89538 A (Daikin Industries, Ltd.), 10 May 2012 (10.05.2012), entire text; particularly, claims; general formula (1) (Family: none) | 1-9 |
| A | WO 2009/063785 A1 (Sumitomo Chemical Co., Ltd.), 22 May 2009 (22.05.2009), entire text; particularly, claims; formula (1), compounds; paragraphs [0020] to [0022], illustrative compounds & JP 2009-137932 A | 1-9 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 05 August, 2014 (05.08.14) | 12 August, 2014 (12.08.14) |

| Name and mailing address of the ISA/ | Authorized officer |
|---|---|
| Japanese Patent Office | |
| Facsimile No. | Telephone No. |

Form PCT/ISA/210 (second sheet) (July 2009)

**INTERNATIONAL SEARCH REPORT**

International application No.
PCT/JP2014/063127

C (Continuation). DOCUMENTS CONSIDERED TO BE RELEVANT

| Category* | Citation of document, with indication, where appropriate, of the relevant passages | Relevant to claim No. |
|---|---|---|
| A | WO 2010/005094 A1 (Sumitomo Chemical Co., Ltd.), 14 January 2010 (14.01.2010), entire text; particularly, claims; page 7, compound examples & JP 2010-41022 A & US 2011/0108884 A1 & EP 2302713 A1 & CN 102084513 A & KR 10-2011-0038110 A | 1-9 |
| A | LEO,L. et al, LANGMUIR, 2000, Vol.16, pp.4599-4606, entire text, particularly, compounds 4, 6 | 1-9 |
| A | JP 2011-140480 A (Daikin Industries, Ltd.), 21 July 2011 (21.07.2011), entire text; particularly, claims (Family: none) | 1-9 |
| A | KOOISTRA,F.B. et al, ORGANIC LETTERS, 2007, Vol.9, No.4, pp.551-554, entire text | 1-9 |
| P,A | US 2014/0024839 A1 (RICOH CO., LTD.), 23 January 2014 (23.01.2014), entire text; particularly, claims (Family: none) | 1-9 |

Form PCT/ISA/210 (continuation of second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2009084264 A
- JP 2010092964 A
- JP 2012089538 A
- JP 2011181719 A


### Non-patent literature cited in the description


- **T. ITOH et al.** *Journal of Materials Chemistry,* 2010, vol. 20, 9, , 226
- **T. OHNO et al.** *Tetrahedron,* 2010, vol. 66, 7, , 316
- **Y. LI et al.** *Journal of American Chemical Society,* 2010, vol. 132, 1, , 377
- **E.T. HOKE et al.** *Advanced Energy Materials,* 2013, vol. 3, 220
- **BROOKE, G.M. et al.** *Tetrahedron,* 1971, vol. 27, 5, , 653
- **ITO et al.** *Journal of Materials Chemistry,* 2010, vol. 20, 9, , 226
- **HUMMELEN et al.** *Organic Letters,* 2007, vol. 9, 551
- **TROSHIN et al.** *Advanced Functional Materials,* 2009, vol. 19, 779